# EUROPEAN PATENT SPECIFICATION

(11) **EP 3 533 471 B1**
(45) Date of publication and mention of the grant of the patent: **19.01.2022**
(21) Application number: 17863851.6
(22) Date of filing: 31.10.2017
(51) Int. Cl.: A61K 48/00

(54) **COMPOSITION FOR ALLEVIATING OR TREATING PAIN**
ZUSAMMENSETZUNG ZUR LINDERUNG ODER BEHANDLUNG VON SCHMERZEN
COMPOSITION POUR LE SOULAGEMENT OU LE TRAITEMENT DE LA DOULEUR

(30) Priority: 31.10.2016 KR 20160143519
(43) Date of publication of application: 04.09.2019
(73) Proprietor: Kolon Life Science, Inc., Seoul 07793 (KR)
(72) Inventor: KIM, Sujeong, Seoul 07062 (KR); CHOI, Heonsik, Seoul 07030 (KR); KWON, Yejin, Seoul 07216 (KR); KIM, Minjung, Seoul 02034 (KR); KIM, Minju, Seoul 03382 (KR); KIM, Daewook, Yongin-si Gyeonggi-do 17111 (KR); PARK, Jangjoon, Seoul 07253 (KR); CHO, Jongho, Seoul 01673 (KR); LEE, Soondong, Gwangmyeong-si Gyeonggi-do 14247 (KR); KIM, Joonsung, Suwon-si Gyeonggi-do 16418 (KR); SIM, Yeomoon, Seoul 05301 (KR)
(74) Representative: HGF
(86) International application number: PCT/KR2017/012136
(87) International publication number: WO 2018/080277

(56) References cited:
- EP-A1- 2 258 841
- WO-A1-2017/052160
- KR-A- 20080 007 968
- KR-A- 20150 080 033
- US-A1- 2007 207 124
- US-A1- 2008 051 357
- US-A1- 2008 051 357
- LIU J ET AL: "Peripherally Delivered Glutamic Acid Decarboxylase Gene Therapy for Spinal Cord Injury Pain", MOLECULAR THERAPY : THE JOURNAL OF THE AMERICAN SOCIETY OF GENE THERAPY, CELL PRESS, US, vol. 10, no. 1, 1 July 2004 (2004-07-01), pages 57-66, XP004660537, ISSN: 1525-0016, DOI: 10.1016/J.YMTHE.2004.04.017
- ERIN D. MILLIGAN ET AL: "Spinal Interleukin-10 Therapy to Treat Peripheral Neuropathic Pain : INTRATHECAL IL-10 THERAPY", NEUROMODULATION, vol. 15, no. 6, 1 June 2012 (2012-06-01), pages 520-526, XP055679242, US ISSN: 1094-7159, DOI: 10.1111/j.1525-1403.2012.00462.x
- HONGWEI YU ET AL: "Analgesia for neuropathic pain by dorsal root ganglion transplantation of genetically engineered mesenchymal stem cells: initial results", MOLECULAR PAIN, BIOMED CENTRAL, LONDON, GB, vol. 11, no. 1, 12 February 2015 (2015-02-12), page 5, XP021210691, ISSN: 1744-8069, DOI: 10.1186/S12990-015-0002-9
- JEAN-MARC G GUEDON ET AL: "Current gene therapy using viral vectors for chronic pain", MOLECULAR PAIN, BIOMED CENTRAL, LONDON, GB, vol. 11, no. 1, 13 May 2015 (2015-05-13), page 27, XP021223165, ISSN: 1744-8069, DOI: 10.1186/S12990-015-0018-1
- Darren Wolfe: "Translating Gene Therapy for Pain from Animal Studies to the Clinic" In: Tuszynski, M.H.: "Translational Neuroscience: Fundamental Approaches for Neurological Disorders", 9 March 2016 (2016-03-09), Springer, New York USA, XP009514844, ISBN: 978-1-4899-7652-9 pages 167-183, DOI: 10.1007/978-1-4899-7654-3_10, * Chapter 10; (eBook: 978-1-4899-7654-3) *
- DATABASE NCBI 7 November 2015 (2015-11-07), "Glutamate Decarboxylase 2 (Homo sapiens)", Database accession no. NCBI reference sequence: NP_000809.1
- DATABASE NCBI 10 August 2014 (2014-08-10), "Interleukin-10 Precursor (Rattus norvegicus)", Database accession no. NP_036986.2
- DATABASE NCBI 15 March 2015 (2015-03-15), "Glial Cell Line-derived Neurotrophic Factor Isoform 2 Preproprotein (Homo sapiens)", Database accession no. NP_954701.1
- Boucher, T.J. et al.: "Potent Analgesic Effects of GDNF in Neuropathic Pain States", Science, vol. 290, no. 5489, 6 October 2000 (2000-10-06), pages 124-127, XP002346300, DOI: 10.1126/science.290.5489.124

## Description

### Technical Field

The present invention relates to a composition for alleviating or treating pain and a method for alleviating or treating pain using the same.

### Background Art

Pain means an experience of actual or potential tissue damage or unpleasant sensations and feelings associated with such damage. Pain protects parts of the body that have been damaged during the healing of the damaged tissues from the damaged situation and provides motivation to avoid similar experiences in the future. Most pain is alleviated slowly when the causal stimulus is removed, but sometimes pain persists even though the tissues have been healed as the stimulus has disappeared and the damage has clearly healed, or pain occurs in a state without any irritation, damage or disease.

For the treatment of pain, mainly, narcotic analgesics such as morphine, which is an opioid alkaloid, or non-narcotic analgesics such as non-steroidal anti-inflammatory drugs (NSAIDs) having the ingredient of acetylsalicylic acid, ibuprofen, or acetaminophen are widely used.

Narcotic analgesics have the advantage of showing the dose-response and high efficacy, but they can lead to nervous system side effects and if used for a long period, they can lead to the resistance and physical dependence, and pain may worsen.

If aspirin, a non-narcotic analgesic having acetylsalicylic acid as a main ingredient, is used for an analgesic purpose, it should be administered at a high dose of at least 500 mg. However, aspirin is a non-steroidal anti-inflammatory analgesic that blocks the enzyme (COX-1) that promotes the production of prostaglandins, which protect the stomach, thereby preventing gastric mucosa formation. Therefore, the stomach may be easily damaged by gastric acid and gastrointestinal bleeding may occur. In addition, ibuprofen is also a non-steroidal anti-inflammatory analgesic, which can cause gastric disturbances. Also, in the case of analgesics having acetaminophen as a main ingredient, such as Tylenol, acetaminophen is mostly metabolized in the liver, and liver damage may be induced.

Even if the above analgesics are effective at an early stage, they often become ineffective due to resistance when used for a long period. Specifically, in the case of neuropathic pain, there is a problem that the pain is non-responsive to the maximum dose of a nonsteroidal anti-inflammatory agent, and thus, it is administered at a high dose for a short period.

Recently, new therapeutic agents for neuropathic pain have been developed, but still have side effects. For example, sodium channel blockers are mostly in the form of small molecules and show low selectivity for isoform proteins. In addition, they show side effects such as cardiac toxicity and movement disorder.

Therefore, there is an imperative need to develop a new analgesic for neuropathic pain which is excellent in analgesic efficacy while reducing side effects.

### Disclosure of Invention

### Technical Problem

Accordingly, the present inventors have endeavored to develop a new analgesic for neuropathic pain exhibiting an excellent analgesic efficacy even at a low dosage. As a result, the present inventors have found that when a combination of two or more of glutamate decarboxylase, an anti-inflammatory cytokine, and a glial cell-derived neurotrophic factor is used, pain can be significantly alleviated or treated as compared with an individual use, and have completed the present invention.

### Solution to Problem

The present invention provides a pharmaceutical composition for alleviating or treating pain comprising two or more selected from the group consisting of a gene encoding glutamate decarboxylase (GAD), a gene encoding interleukin-10 (IL-10), and a gene encoding a glial cell-derived neurotrophic factor (GDNF).

### Advantageous Effects of Invention

A pharmaceutical composition of the present invention comprises two or more selected from the group consisting of genes encoding GAD, IL-10, and GDNF. Therefore, the pharmaceutical composition of the present invention exhibits an excellent analgesic efficacy at a dosage lower than that of individual administration since genes are co-administered, and thus conventional side effects and toxicity can be reduced. Therefore, the pharmaceutical composition of the present invention can be useful in alleviating or treating pain.

### Brief Description of Drawings

Fig. 1 shows the schematic diagram of the plasmids pAAV-GAD65 and pAAV-GAD65-modi used for the construction of the recombinant adeno-associated virus:
   (a) shows the schematic diagram of pAAV-GAD65, and (b) shows the schematic diagram of pAAV-GAD65-modi.
Fig. 2 shows the schematic diagram of the plasmid pAAV-IL-10 used for the construction of the recombinant adeno-associated virus.
Fig. 3 shows the schematic diagram of the plasmid pAAV-GDNF used for the construction of the recombinant adeno-associated virus.
Fig. 4 is a schematic diagram showing the pAAV-GDNF-IL-10 plasmid.
Fig. 5 shows the expression of each introduced gene by the pAAV-GAD65, pAAV-IL-10, or pAAV-GDNF plasmid:
   (a) shows the expression of GAD65 by pAAV-GAD65 plasmid; (b) shows the expression of IL-10 by pAAV-IL-10 plasmid; and (c) shows the expression of GDNF by the pAAV-GDNF plasmid.
Fig. 6 shows the expression of GDNF gene and IL-10 gene by the pAAV-GDNF-IL-10 plasmid:
   (a) shows the expression of IL-10 by pAAV-GDNF-IL-10 plasmid; and (b) shows the expression of GDNF by pAAV-GDNF-IL-10 plasmid.
Fig. 7 shows the results of Western blot showing expression of each protein after treatment of 293T or HeLa cells with each recombinant adeno-associated virus after construction of the recombinant adeno-associated viruses into which GAD65 gene, IL-10 gene and GDNF gene were introduced, respectively:
   (a) shows the expression of GAD65 after treatment of 293T or HeLa cells with the recombinant adeno-associated virus AAV-GAD65; (b) shows the expression of GAD65 after treatment of 293T or HeLa cells with the recombinant adeno-associated virus AAV-GAD65-modi; (c) shows the expression of IL-10 after treatment of 293T or HeLa cells with the recombinant adeno-associated virus AAV-IL-10; and (d) shows the expression of GDNF after treatment of 293T or HeLa cells with the recombinant adeno-associated virus AAV-GDNF.

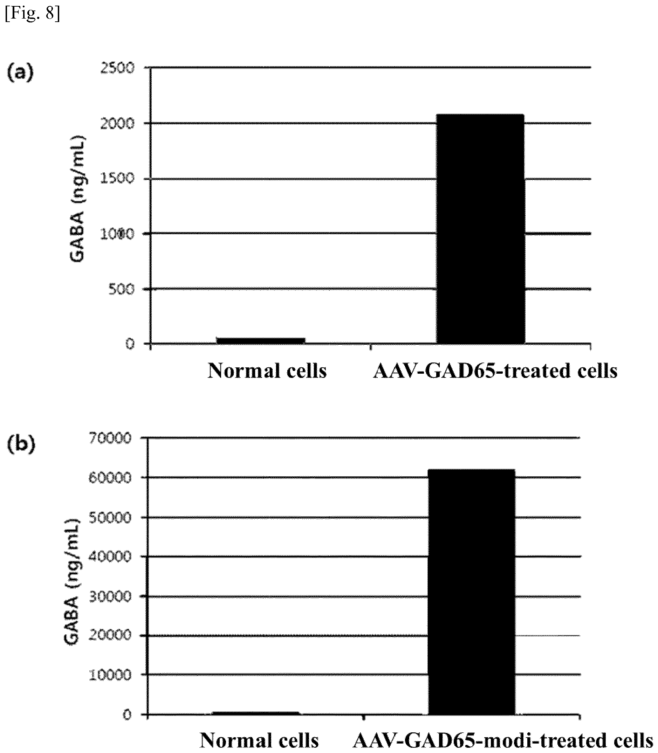

Fig. 8 shows the levels of GABA expression measured by ELISA after treatment of 293T or HeLa cells with the recombinant adeno-associated virus AAV-GAD65 or AAV-GAD65-modi:
   (a) is a graph showing the level of GABA expression after 293T or HeLa cells were treated with the recombinant adeno-associated virus AAV-GAD65; and (b) is a graph showing the level of GABA expression after 293T or HeLa cells were treated with the recombinant adeno-associated virus AAV-GAD65-modi.

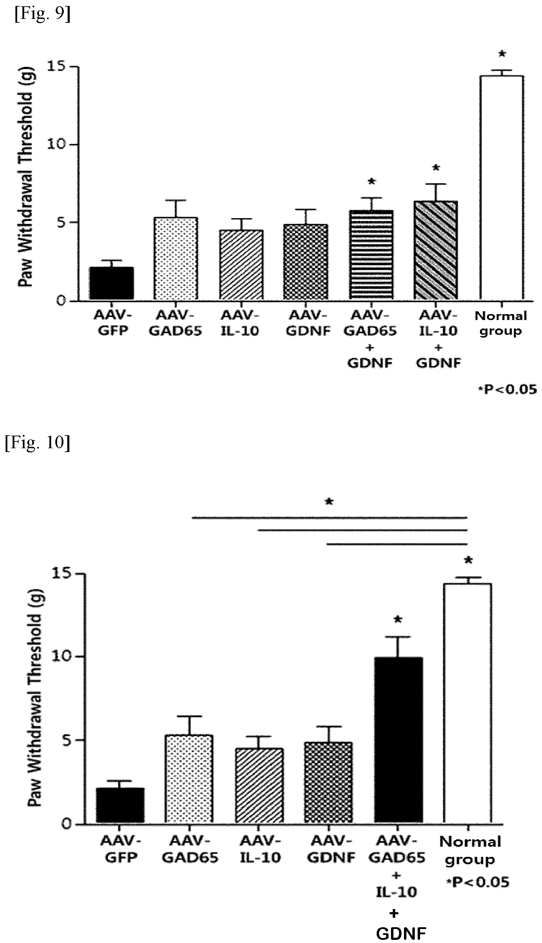

Fig. 9 is a graph showing the results of comparing the pain alleviating efficacies between individual administration of AAV-GAD65, AAV-IL-10, or AAV-GDNF virus and co-administration of AAV-GAD65 and AAV-GDNF viruses, or AAV-IL-10 and AAV-GDNF viruses.
Fig. 10 is a graph showing the results of comparing the pain alleviating efficacies between individual administration of AAV-GAD65, AAV-IL-10, or AAV-GDNF virus and co-administration of AAV-GAD65 , AAV-IL-10 and AAV-GDNF virus.

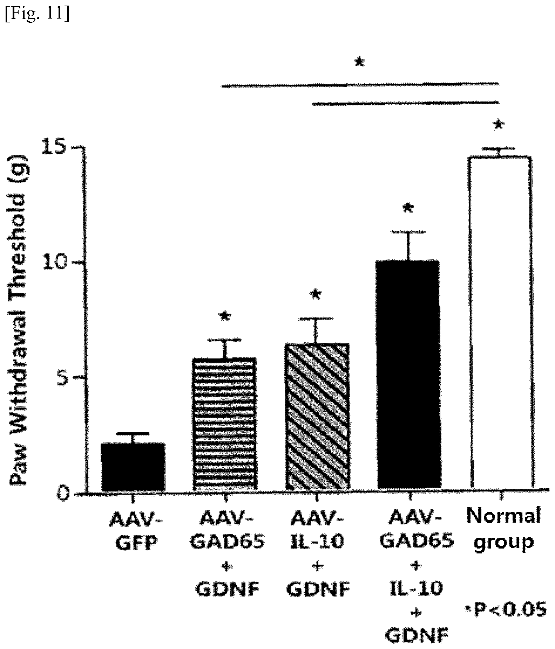

Fig. 11 is a graph showing the results of comparing the pain alleviating efficacies between co-administration of AAV-GAD65 and AAV-GDNF viruses, or AAV-IL-10 and AAV-GDNF viruses and co-administration of all of the AAV-GAD65, AAV-IL-10 and AAV-GDNF viruses.

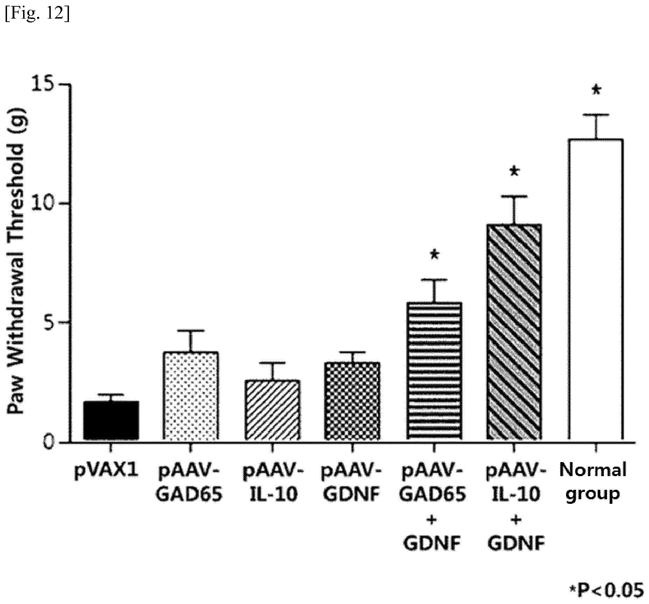

Fig. 12 is a graph showing the results of comparing the pain alleviating efficacies between individual administration of pAAV-GAD65, pAAV-IL-10, or pAAV-GDNF plasmid and co-administration of pAAV-GAD65 and pAAV-GDNF plasmids, or pAAV-IL-10 and pAAV-GDNF plasmids.

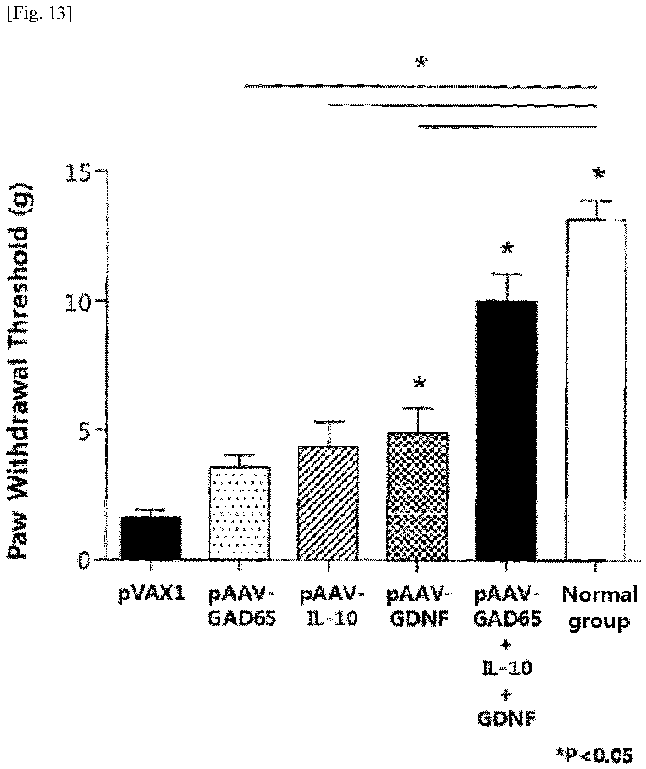

Fig. 13 is a graph showing the results of comparing the pain alleviating efficacies between individual administration of pAAV-GAD65, pAAV-IL-10, or pAAV-GDNF plasmid and co-administration of all of the pAAV-GAD65, pAAV-IL-10 and pAAV-GDNF plasmids.

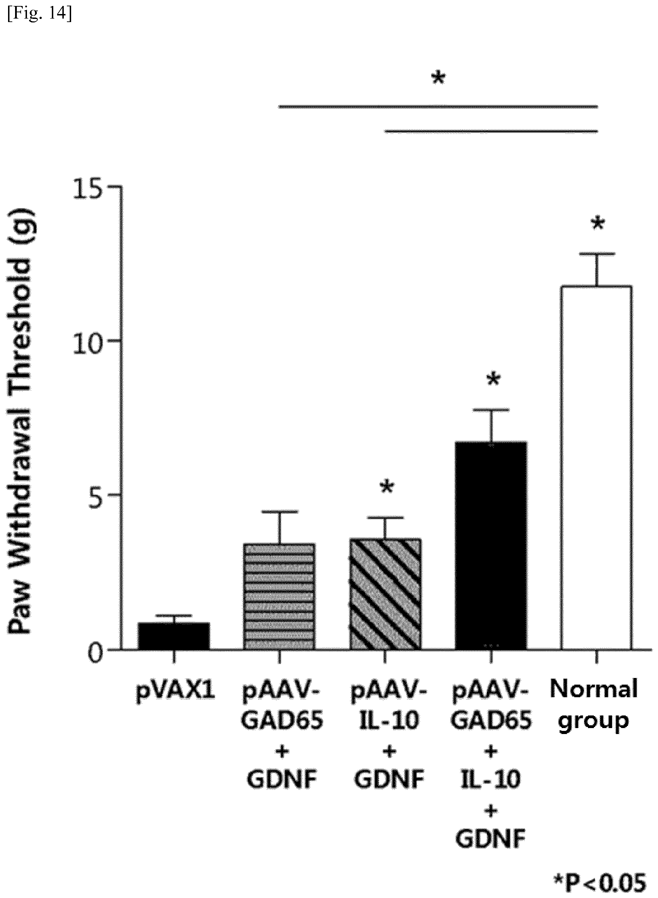

Fig. 14 is a graph showing the results of comparing the pain alleviating efficacies between co-administration of pAAV-GAD65 and pAAV-GDNF plasmids, or pAAV-IL-10 and pAAV-GDNF plasmids and co-administration of all of the pAAV-GAD65, pAAV-IL-10 and pAAV-GDNF plasmids.

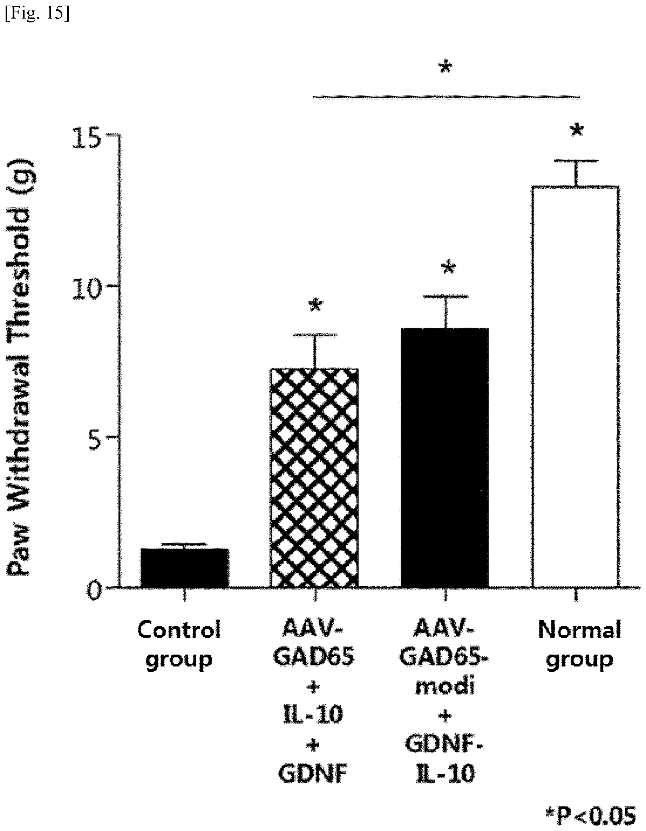

Fig. 15 is a graph showing the results of comparing the pain alleviating efficacies between co-administration of AAV-GAD65-modi and AAV-GDNF-IL-10 viruses and co-administration of all of the AAV-GAD65, AAV-IL-10 and AAV-GDNF viruses.

### Best Mode for Carrying out the Invention

Hereinafter, the present invention will be described in detail.

The present invention provides a pharmaceutical composition for alleviating or treating pain comprising two or more selected from the group consisting of a gene encoding glutamate decarboxylase (GAD), a gene encoding interleukin-10 (IL-10), and a gene encoding a glial cell-derived neurotrophic factor (GDNF).

In one embodiment, the combination of two or more may be GAD and IL-10, GAD and GDNF, IL-10 and GDNF, or GAD, IL-10 and GDNF.

Two or more genes selected from the group consisting of a gene encoding GAD, a gene encoding IL-10, and a gene encoding GDNF may be in a form of being contained in a carrier. Herein, the carrier may be a viral vector, or a non-viral vector such as a plasmid, a liposome, etc. In addition, the genes may be in a form in which some of the genes are contained in a viral vector and the remaining genes are contained in a non-viral vector.

In one embodiment, the genes may be in a form in which GAD is contained in a viral vector, and IL-10 is contained in a non-viral vector. In addition, the genes may be in a form in which GAD is contained in a viral vector, and GDNF is contained in a non-viral vector. Further, the genes may be in a form in which IL-10 is contained in a viral vector, and GDNF is contained in a non-viral vector. In addition, the genes may be in a form in which GAD is contained in a viral vector, and IL-10 and GDNF are contained in a non-viral vector. In addition, the genes may be in a form in which GAD and IL-10 are contained in a viral vector, and GDNF is contained in a non-viral vector. In addition, the genes may be in a form in which GAD and GDNF are contained in a viral vector, and IL-10 is contained a non-viral vector. In addition, the genes may be in a form in which IL-10 is contained in a viral vector, and GAD and GDNF are contained in a non-viral vector. In addition, the genes may be in a form in which IL-10 and GDNF are contained in a viral vector, and GAD is contained in a non-viral vector. Also, the genes may be in a form in which GDNF is contained in a viral vector, and GAD and IL-10 are contained in a non-viral vector.

In addition, the gene may be in a form of being operably contained in a vector. Specifically, the gene may be in a form of being operably contained in a viral vector or a non-viral vector.

The viral vector may be at least one selected from the group consisting of adenovirus, adeno-associated virus (AAV), herpes simplex virus, lentivirus, retrovirus, cytomegalovirus, baculovirus, poxvirus, etc. Specifically, the viral vector may be adeno-associated virus.

In one embodiment, the gene encoding GAD may be operably contained in a carrier 1 (e.g., a first vector), and the gene encoding IL-10 may be operably contained in a carrier 2 (e.g., a second vector), and the gene encoding GDNF may be operably contained in a carrier 3 (e.g., a third vector). In addition, one carrier may contain two or more genes.

The non-viral vector may be at least one selected from the group consisting of a plasmid, a liposome, a cationic polymer, a micelle, an emulsion, and solid lipid nanoparticles.

The term "plasmid" as used herein refers to a circular DNA fragment existing separately outside the chromosome of bacteria. Plasmids have no genes essential for the survival of bacteria, but contain genes essential for resistance to certain antibiotics and for interbacterial gene exchange. In addition, plasmids can grow independently of chromosomes and contain selectable markers.

The term "liposome" as used herein refers to a small vesicle produced by forming a bilayer due to the hydrophilic portion and the hydrophobic portion when a molecule having both a hydrophobic portion and a hydrophilic portion in a molecule, such as a phospholipid, is suspended in an aqueous solution. Liposome is isolated from the outer membrane by a membrane composed of a lipid bilayer, and liposomes containing DNA, mRNA, etc., can be used as mediators of genetic information.

The term "cationic polymer" as used herein refers to a cationic lipid or a polymer compound which is a substance that forms a complex by an ionic bond with anionic DNA and delivers the DNA into a cell.

The term "micelle" as used herein refers to a thermodynamically stable colloidal aggregate formed from the molecules consisting of a polar group and a nonpolar hydrophobic group, such as surfactants and lipid molecules, through association by a van der Waals force or the like in a solution. In addition, micelles containing DNA, mRNA and the like can be used as mediators of genetic information.

The term "emulsion" as used herein means that, when two solutions of different phases are mixed, one liquid forms fine particles and is dispersed in another liquid. DNA, mRNA and the like may be contained in the center of the emulsion particle to be used as mediators of genetic information.

As used herein, the term "solid lipid nanoparticle" refers to a preparation of a form in which a drug is contained in a nano-sized microparticle made of a solid lipid instead of a liquid lipid.

A carrier 1 (e.g., a first vector) comprising any one gene selected from the group consisting of GAD, IL-10, and GDNF, and a carrier 2 (e.g., a second vector) comprising any one gene selected from the remaining gene group not included in the carrier 1 according to the present invention may have a virus titer-based mixing ratio per unit volume of 1: 1 to 100 or 1 to 100: 1. Specifically, the virus titer-based mixing ratio per unit volume of the carrier 1 and the carrier 2 may be 1: 1 to 10 or 1 to 10: 1.

A carrier 1 (e.g., a first vector) comprising a gene encoding GAD, a carrier 2 (e.g., a second vector) comprising a gene encoding IL-10 and a carrier 3 (e.g., a third vector) comprising a gene encoding GDNF according to the present invention may have a virus titer-based mixing ratio per unit volume of 1: 0.1 to 10: 0.1 to 10.

As used herein, the term "operably" means that an introduced gene is linked to a regulatory sequence in such a way that expression can take place in a host cell. The regulatory sequence is a DNA sequence that regulates the expression of the gene, and may include other regulatory elements such as promoters and enhancers or polyadenylation. In addition, the regulatory sequence provides a site for binding of a transcription factor that controls the expression of the introduced gene, and can influence the complex structure with the transcription factor to determine the function of the transcription factor.

The term "GAD" as used herein refers to an enzyme that decarboxylates glutamate to produce GABA (gamma-aminobutyric acid). The GAD may be GAD65 or GAD67. Specifically, GAD65 may be derived from a human, a rat, a dog, a cat, or a horse, but is not limited thereto. The gene encoding GAD may be the nucleotide sequence encoding the amino acid sequence represented by SEQ ID NO: 1, 4, 32, 34, or 36.

In addition, the nucleotide sequence encoding the amino acid sequence represented by SEQ ID NO: 1 may be the DNA sequence represented by SEQ ID NO: 2 or 3, and may be the mRNA sequence shown in NCBI Reference Sequence: NM_000818.2. In addition, the nucleotide sequence encoding the amino acid sequence represented by SEQ ID NO: 4 may be the nucleotide sequence which was codon-optimized to be suitable for the DNA sequence represented by SEQ ID NO: 5 or the gene encoding the amino acid sequence represented by SEQ ID NO: 4, which may be the mRNA sequence shown in NCBI Reference Sequence: NM_000817.2. In addition, the nucleotide sequence encoding the amino acid sequence represented by SEQ ID NO: 32 may be the DNA sequence represented by SEQ ID NO: 33. The nucleotide sequence encoding the amino acid sequence represented by SEQ ID NO: 34 may be the DNA sequence represented by SEQ ID NO: 35, and the nucleotide sequence encoding the amino acid sequence represented by SEQ ID NO: 36 may be the DNA sequence represented by SEQ ID NO: 37.

In addition, the gene encoding GAD may be a nucleotide sequence encoding a GAD variant which can retain GAD activity and produce GABA. The GAD variant includes all sequences which retain GAD's characteristics of producing GABA. Although not limited to any one sequence, the nucleotide sequence encoding the GAD variant may be, preferably, a nucleotide sequence encoding an amino acid sequence having a sequence homology of at least 60% or more, 70% or more, 80% or more, or 90% or more, and to the GAD's amino acid sequence described above, and most preferably, may be a nucleotide sequence encoding an amino acid sequence having a sequence homology of 95% or more.

In addition, the nucleotide sequence encoding the GAD variant may be a nucleotide sequence having a sequence homology of at least 60% or more, 70% or more, 80% or more, or 90% or more to the GAD nucleotide sequence described above, and most preferably, may be a nucleotide sequence having a sequence homology of 95% or more.

The "% of sequence homology" is determined by comparing the comparison regions in a state in which two sequences are optimally aligned. In addition, some of the nucleotide sequences in the comparison regions may include additions or deletions (i.e., gaps) relative to the reference sequence (without addition or deletion) for the optimal alignment of the two sequences.

The term "IL-10" as used herein refers to an anti-inflammatory cytokine belonging to the class II cytokine (Renauld, Nat Rev Immunol, 2003). The IL-10 is in a form of a homodimer consisting of two subunits each of which has the length of 178 amino acids. It is also known as the cytokine synthesis inhibitory factor (CSIF) in humans. IL-10 serves the function of inhibiting the activity of NK (natural killer) cells in the immune response, and forms a complex with an IL-10 receptor to be involved in signal transduction. IL-10 may be a protein derived from a human, a rat, a dog, a cat, or a horse, but is not limited thereto. The gene encoding IL-10 may be the nucleotide sequence encoding the amino acid sequence represented by SEQ ID NO: 6, 9, 38, 40, or 42.

Specifically, the nucleotide sequence encoding the amino acid sequence represented by SEQ ID NO: 6 may be the DNA sequence represented by SEQ ID NO: 7 or 8, and may be the mRNA sequence shown in NCBI Reference Sequence: NM_012854.2. In addition, the nucleotide sequence encoding the amino acid sequence represented by SEQ ID NO: 9 may be the DNA sequence represented by SEQ ID NO: 10 or 14, and may be the mRNA sequence shown in NCBI Reference Sequence: NM_000572.2. The nucleotide sequence encoding the amino acid sequence represented by SEQ ID NO: 38 may be the DNA sequence represented by SEQ ID NO: 39. In addition, the nucleotide sequence encoding the amino acid sequence represented by SEQ ID NO: 40 may be the DNA sequence represented by SEQ ID NO: 41, and the nucleotide sequence encoding the amino acid sequence represented by SEQ ID NO: 42 may be the DNA represented by SEQ ID NO: 43.

In addition, the gene encoding IL-10 may be a nucleotide sequence encoding an IL-10 variant that retains the activity of IL-10. The nucleotide sequence encoding the IL-10 variant may be a nucleotide sequence encoding an amino acid sequence having a sequence homology of at least 60% or more, 70% or more, 80% or more, 90% or more to the IL-10 amino acid sequence shown above, and most preferably, may be a nucleotide sequence encoding an amino acid sequence having a sequence homology of 95% or more.

The nucleotide sequence encoding the IL-10 variant may be a nucleotide sequence having a sequence homology of at least 60% or more, 70% or more, 80% or more, 90% or more to the IL-10 nucleotide sequence shown above, and most preferably, may be a nucleotide sequence having a sequence homology of 95% or more.

The term "GDNF" as used herein refers to a protein constituting the GDNF ligand family. The GDNF ligand family consists of GDNF, neurturin (NRTN), artemin (ARTN), and persephin (PSPN). In addition, GDNF is a protein that promotes the survival of many kinds of neurons and transmits signals through the GFRα1 receptor. GDNF may be a protein derived from a human, a rat, a dog, a cat, or a horse, but is not limited thereto. The gene encoding GDNF may be the nucleotide sequence encoding the amino acid sequence represented by SEQ ID NO: 11, 44, 46, or 48.

Specifically, the nucleotide sequence encoding the amino acid sequence represented by SEQ ID NO: 11 may be the DNA sequence represented by SEQ ID NO: 12 or 13, and may be the mRNA sequence shown in NCBI Reference Sequence: NM_199231.2. In addition, the nucleotide sequence encoding the amino acid sequence represented by SEQ ID NO: 44 may be the DNA sequence represented by SEQ ID NO: 45. The nucleotide sequence encoding the amino acid sequence represented by SEQ ID NO: 46 may be the DNA sequence represented by SEQ ID NO: 47, and the nucleotide sequence encoding the amino acid sequence represented by SEQ ID NO: 48 may be the DNA sequence represented by SEQ ID NO: 49.

In addition, the gene encoding GDNF may be a nucleotide sequence encoding a GDNF variant which retains the GDNF activity. The nucleotide sequence encoding the GDNF variant may be a nucleotide sequence encoding an amino acid sequence having a sequence homology of at least 60% or more, 70% or more, 80% or more, or 90% or more to the GDNF's amino acid sequence shown above, and most preferably, may be a nucleotide sequence encoding an amino acid sequence having a sequence homology of 95% or more.

In addition, the nucleotide sequence encoding the GDNF variant may be a nucleotide sequence having a sequence homology of at least 60% or more, 70% or more, 80% or more, 90% or more to the GDNF nucleotide sequence shown above, and most preferably, may be a nucleotide sequence having a sequence homology of 95% or more.

GABA, the product of GAD gene, has the effect of blocking pain signal transduction, but excessive amounts can cause symptoms such as itching, dizziness, drowsiness, etc., as well as the side effects such as increase in the heart rate or respiratory rate (Longo, Am Fam Physician, 2000).

IL-10 is known to be a cytokine which shows anti-inflammatory actions, but side effects such as flu symptoms and the like can occur (Friedrich, J Invest Dermatol, 2002).

Furthermore, it is known that the expression of GDNF exhibits analgesic efficacies on a variety of pains such as neuropathic pain and the like, but it has been reported in monkey experiments that administration in excess caused neuronal damage of brain (Hovland, Toxicol Pathol, 2007).

A pharmaceutical composition of the present invention can exhibit analgesic actions with a small amount of genes or carriers containing the same. The composition of the present invention consists of a vector containing a gene encoding GAD, a vector containing a gene encoding an anti-inflammatory cytokine in nervous tissues, and/or a vector containing a gene encoding GDNF. And by co-administering substances having different analgesic mechanisms, it is possible to achieve the same or better pain alleviation or treatment effects at a dosage lower than that of individual administration.

Particularly, according to the present invention, when two or more genes selected from the group consisting of genes encoding GAD65, IL-10, and GDNF are co-administered, a synergistic pain-alleviating effect takes place. Therefore, the pharmaceutical composition of the present invention can be useful for alleviating or treating pain.

According to one embodiment of the present invention, the first vector, the second vector, and/or the third vector may be an adeno-associated virus. The adeno-associated virus is not limited to a particular serotype, and preferably may be any one of AAV1 to AAV9.

The pain may be selected from the group consisting of nociceptive pain, psychogenic pain, inflammatory pain, pathological pain, neuropathic pain, cancer pain, postoperative pain, trigeminal neuralgia pain, idiopathic pain, diabetic neuropathic pain, or migraine. In a specific example, the pain may be lumbosacral radiculopathy (LSR).

The inflammatory pain refers to the pain associated with a tissue damage and infiltration of immune cells. In addition, the pathological pain means a disease state in which pain is caused by damage to a nerve tissue or its abnormal function. Also, the pathological pain may be dysfunctional pain, such as fibromyalgia, irritable bowel syndrome, or tension headache.

In addition, pain can include back pain which can be anatomically distinguished: neck pain, middle back pain, lower back pain, or tailbone pain. In addition, the pain may be at least one selected from the group consisting of neuropathic pain, cancer pain, postoperative pain, trigeminal neuralgia pain, idiopathic pain, diabetic neuropathic pain, migraine, and the like. In a specific example, the pain may be lumbosacral radiculopathy.

Neuropathic pain can be caused by a damage or disease that affects the somatosensory system. Neuropathic pain can be an abnormal sensation called allodynia and dysesthesia. In addition, the general characteristics of neuropathic pain include the sense of hot or cold, pins and needles, numbness, and itching. In contrast, nociceptive pain is often expressed as aching.

In addition, migraine is associated with a number of autonomic nervous system symptoms, and is a chronic disorder that causes headaches of normal to serious severities. Migraine is known to be associated with increased excitability of the cerebral cortex and abnormal regulation of pain neurons in the trigeminal nucleus of the brainstem (Noseda, Pain, 2013).

Specifically, a pharmaceutical composition of the present invention can be used for alleviating or treating neuropathic pain and chronic cancer pain.

As used herein, the term "alleviating or treating" means any action that improves or alters pain symptom in a beneficial way by administering the composition of the present invention.

The pharmaceutical composition of the present invention may further comprise a physiologically acceptable carrier. In addition, the pharmaceutical compositions of the present invention may further comprise suitable excipients and diluents conventionally used in the preparation of pharmaceutical compositions. In addition, the compositions of the present invention may be used by preparing them as oral formulations such as powders, granules, tablets, capsules, suspensions, emulsions, syrups, aerosols, etc., external formulations, suppositories, or injections by general methods. Specifically, the pharmaceutical composition may be in the form of an injection. As for the suitable formulations known in the art, those listed in Remington's Pharmaceutical Science (1985) may be used.

In addition, the pharmaceutical composition may comprise a salt (sodium chloride), lactose, dextrose, sucrose, sorbitol, mannitol, xylitol, erythritol, maltitol, starch, acacia gum, alginate, gelatin, calcium phosphate, calcium silicate, cellulose, methylcellulose, microcrystalline cellulose, polyvinylpyrrolidone, water, methyl hydroxybenzoate, propyl hydroxybenzoate, talc, magnesium stearate, mineral oil, etc., as carriers, excipients, and diluents. For the formulation of the pharmaceutical composition of the present invention, generally used diluents or excipients such as fillers, extenders, binders, humectants, disintegrators, surfactants, etc. may be utilized.

Formulations for parenteral administration may include sterile solutions, non-aqueous solvents, suspensions, emulsions, freeze-dried formulations, and suppositories. Propylene glycol, polyethylene glycol, vegetable oil such as olive oil, and injectable ester such as ethylolate, etc., may be used for non-aqueous solvents and suspensions. Witepsol, macrogol, Tween 61, cacao oil, laurin oil, glycerogelatin, etc. may be used for suppository bases.

The pain is as described above with regard to the pharmaceutical composition.

The subject may be a mammal including a human, or a cell and/or tissue isolated from a mammal including a human. The term "non-human animal" as used herein is intended to encompass all vertebrate animals, which include mammals and non-mammals such as primates, sheep, dogs, cats, horses, cows, chickens, amphibians, reptiles, etc.

As for the administration route, dosage, and administration frequency, the pharmaceutical composition may be administered to a subject in various ways and amounts depending on the condition of a patient and the presence or absence of side effects, and the range of optimal administration methods, dosages and administration frequencies may be appropriately selected by those having ordinary skill in the art. In addition, the pharmaceutical composition may be administered in combination with another drug or a physiologically active substance which is known to show a therapeutic efficacy on a disorder to be treated. Also, the pharmaceutical composition may be prepared in the form of a combination formulation.

Specifically, the pharmaceutical composition of the present invention may be provided in the form of an injection. For example, subcutaneous injection, intramuscular injection, intravenous injection, epidural injection, or intrathecal injection, and the like may be included. Specifically, the pharmaceutical composition may be administered via epidural injection or intrathecal injection, and more specifically, it may be administered via transforaminal epidural injection or intrathecal injection.

As used herein, the term "transforaminal epidural injection" refers to a method of injecting a drug into the inside of an intervertebral foramen which is a space where nerves emerge from the spinal cord through the space between spinal bones, and into the space outside of the dura which surrounds the spinal cord and spinal nerves. In one embodiment, if the pharmaceutical composition of the present invention is made of viruses, the drug can be administered to the inside of the intervertebral foramen of a subject by conducting epidural injection therapy.

As used herein, the term "intrathecal injection" refers to a method of administration by injecting a drug to a space inside dura in the spinal canal. In one embodiment, if the pharmaceutical composition of the present invention is made of plasmids, the drug can be administered to the inside of the spinal canal of a subject by conducting intrathecal injection therapy.

Specifically, if the pharmaceutical composition is made of viral vectors, it can be administered in an amount of 1.0 × 10⁶ to 1.0 × 10¹⁴ vg on an adult basis. In addition, when there are two types of viruses to be administered, each type of the viruses can be administered in an amount of 5.0 × 10⁵ to 5.0 × 10¹³ vg. If there are three types of viruses to be administered, each type of the viruses can be administered in an amount of 3.0 × 10⁵ to 3.0 × 10¹³ vg.

In addition, if the pharmaceutical composition is made of non-viral vectors, it can be administered in a concentration of 0.1 mg/ml to 10 mg/ml, on an adult basis. Also, if the pharmaceutical composition is made of plasmid vectors, the dosage may be 0.1 ml, 1 ml, 2 ml, 3 ml, 4 ml, 5 ml, 6 ml, 7 ml, 8 ml, 9 ml, 10 ml or more, including all values and ranges between them.

As for the administration frequency of a viral vector, it may be administered once or more, or 1 to 10 times. Also, it may be administered at the interval of 1 day to 1 month, or 1 month to 1 year in the case of repeated administration. In addition, if the pharmaceutical composition is made of non-viral vectors, it may be administered 1 or more, or 1 to 10 times. Also, it may be administered at the interval of 12 to 24 hours or 1 to 14 days in the case of repeatedly administration.

The present invention provides a pharmaceutical composition for the use of alleviating or treating pain.

The present invention provides a use of the pharmaceutical composition of the present invention for preparing a therapeutic agent for alleviating or treating pain.

### Modes for Carrying Out the Invention

Hereinafter, the present invention will be described in detail with reference to examples. However, the following examples are for illustrative purposes only and are not intended to limit the scope of the present invention.

### Example 1. Preparation and property analysis of recombinant adeno-associated virus

Adeno-associated viruses required for the present invention were constructed and produced on the basis of the AAV helper-free system (Agilent).

### Example 1.1. Construction of pAAV-GAD65 plasmid

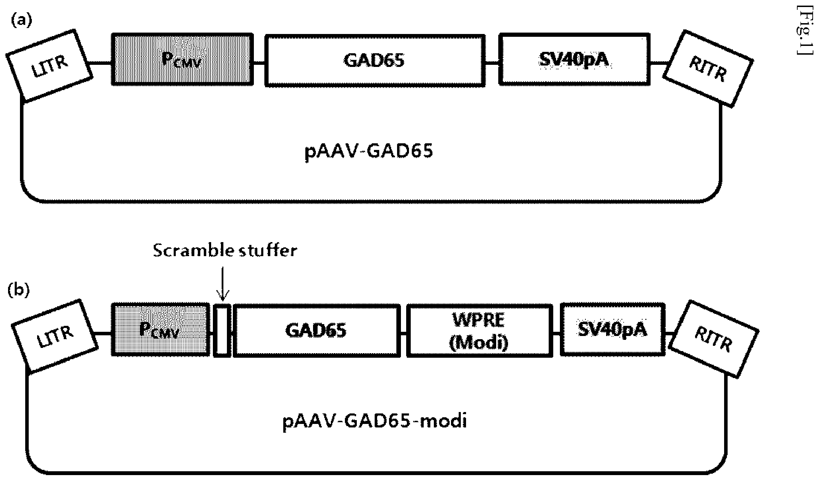

To construct the pAAV-GAD65 plasmid of Fig. 1, the CMV promoter region of pJDK-rGAD65 (Lee, Gene Ther, 2005) was amplified by PCR, and then the resultant was introduced into pGEM-T (Promega) to construct pGEM-T-CMV. The primer sequences used for CMV promoter amplification are as follows:
F-JDK (SEQ ID NO: 15): 5'-TTCGGCCGTCGAGGAGCTTGGCCCATTG-3'
R-JDK (SEQ ID NO: 16): 5'-GACGTCGACCTAGCTAGCGAATTCGGGGCCGCGGAG-3'.

As for the GAD65 gene, the gene represented by SEQ ID NO: 3 was designed by codon-optimization to be suitable for humans based on the human GAD65 (NCBI NM_000818.2) represented by the amino acid sequence of SEQ ID NO: 1, and referred to Bioneer for gene synthesis. The hGAD65 gene introduced into pGEM-T was treated with NheI and SalI to prepare a 1.7 Kb DNA fragment. Thereafter, it was subjected to ligation with the 3.7 Kb DNA fragment obtained by treating pGEM-T-CMV with NheI and SalI, to complete pGEM-T-CMV-hGAD65 construction.

SV40pA was amplified by conducting PCR using pCI (Invitrogen) as a template, and then the resultant was treated with ClaI and SalI to prepare a 222 bp DNA fragment. The DNA fragment was subjected to ligation with the 5.4 Kb DNA fragment prepared by cutting pGEM-T-CMV-hGAD65 with ClaI and SalI, to finally prepare pGEM-T-CMV-hGAD65-SV40pA. The primer sequences used for SV40pA amplification are as follows:
F-SV40pA (SEQ ID NO: 17): 5'-CCATCGATCAGACATGATAAGATACATTGATGAG-3'
R-SV40pA (SEQ ID NO: 18): 5'-GACGTCGACGCGGCCGCTACCACATTTGTAGAGGTTTTACTTG-3'.

To construct an adeno-associated virus vector, the ampicillin resistance gene in pAAV-MCS (Agilent) was replaced with the kanamycin resistance gene. The kanamycin resistance gene was amplified by PCR using pET-28 (a) (Novagen) as a template. The amplified 816 bp kanamycin resistance gene was subjected to ligation with pGEM-T to construct pGEM-T-Kan^{r}. The primer sequences used for kanamycin resistance gene amplification are as follows:
F-Kan (SEQ ID NO: 19): 5'-AGGCGCCATGAGCCATATTCAACGGGAA-3'
R-Kan (SEQ ID NO: 20): 5'-TTCATGATTAGAAAAACTCATCGAGCATC-3'.

To introduce the kanamycin resistance gene, SpeI and EcoRV sites were respectively generated by mutagenesis upstream and downstream of the ampicillin resistance gene in pAAV-MCS, and then the resultant was treated with SpeI and EcoRV again. The resultant was subjected to ligation with the DNA fragment obtained by cutting the previously constructed pGEM-T-Kan^{r} with NheI and EcoRV, to construct pAAV-MCS-Kan^{r}.

The constructed pAAV-MCS-Kan^{r} was treated with NotI and BamHI, and then subjected to ligation with the 2.7 Kb DNA fragment obtained by cutting pGEM-T-CMV-hGAD65-SV40pA with EagI and PvuI, to construct pssAAV-GAD65.

To introduce the GAD65 expression cassette into pVAX1 (Invitrogen), the BamHI site was generated by mutagenesis downstream of the bGHpA. Then, the resultant was cut with MluI and NheI to prepare DNA fragments. The LITR and CMV promoter regions were amplified by PCR using pssaAV-GAD65 as a template and cloned into pGEM-T easy (Promega). Thereafter, the resultant was cut with AscI and NheI, and subjected to ligation with the pVAX1 vector previously prepared, to construct pVAX1-LITR-CMV. Primer sequences used for LITR and CMV promoter region amplification are as follows:
F-ITR (SEQ ID NO: 21): 5'-ATGGCGCGCCCCTGGCCTTTTGCTGGCC-3',
R-JDK (SEQ ID NO: 16): 5'-GACGTCGACCTAGCTAGCGAATTCGGGGCCGCGGAG-3'.

pVAX1-LITR-CMV was cut with NotI and NheI again to prepare DNA fragments. PSSAAV-GAD65 was cut with EagI and NheI, and subjected to ligation with the DNA fragments previously prepared, to construct pVAX1-LITR-CMV-hGAD65-SV40pA.

The pVAX1-LITR-CMV-hGAD65-SV40pA was cut with HpaI and BamHI to prepare DNA fragments. In addition, psA-SV40pA-RITR, which had been prepared by amplifying through PCR using pssaAV-GAD65 as a template and cloning into pGEM-T easy, was treated with HpaI and BamHI, to prepare DNA fragments. The two DNA fragments were ligated to complete pVAX1-LITR-CMV-hGAD65-SV40pA-RITR (hereinafter abbreviated as "pAAV-GAD65"). Primer sequences used for SV40pA and RITR region amplification are as follows:
F-SV40pA (SEQ ID NO: 17): 5'-CCATCGATCAGACATGATAAGATACATTGATGAG-3'
R-ITR (SEQ ID NO: 22): 5'-ATGGATCCGCTAGTAAATACCGCATCAG-3'.

The schematic diagram of the pAAV-GAD65 plasmid is shown in Fig. 1.

The following procedure was carried out to construct modified pAAV-GAD65.

First, the vector was cut with Nhe1, and then an arbitrary random nucleotide sequence was inserted between the CMV promoter and GAD65 gene by an infusion method. The inserted nucleotide sequences are as follows:
Scramble stuffer (SEQ ID NO: 29):
5'-GTCGACGGTATCGATAAGCTTGATATCGAATTCCTGCAGCCC-3'
Stuffer_scramble_F (SEQ ID NO: 30):
Stuffer_scramble_R (SEQ ID NO: 31):

Next, the WPRE nucleotide sequence (Schambach, Gene Ther, 2006), from which the X-protein region which can provide an oncogenic effect was removed, was amplified by PCR, and inserted at the back of GAD65 gene using Pac1 and Hpa1 restriction enzymes. At the same time, some portion of SV40pA was removed to construct a modified SV40pA. The primer sequences used for WPRE amplification are as follows:
WPRE_Pac1_F (SEQ ID NO: 25):
5'-GGTGGTTTAATTAAAATCAACCTCTGGATTACAAAATTTG-3'
WPRE_modi_Hpa1_R (SEQ ID NO: 26): 5'-GGTGGTGTTAACGACAACACCACGGAATTG-3'.

The finally modified plasmid was pVAX1-LITR-CMV-scramble stuffer-hGAD65-WPRE (modi)-SV40pA (modi)-RITR (hereinafter abbreviated as "pAAV-GAD65-modi"), and its schematic diagram is shown in Fig. 1.

### Example 1.2. Construction of pAAV-IL-10 plasmid

The pAAV-IL-10 plasmid was constructed by the same method as in Example 1.1. As for the rat IL-10 gene, the gene represented by the nucleotide sequence of SEQ ID NO: 8 was designed by codon-optimization to be suitable for rats based on the human IL-10 (NCBI NM-012854) represented by the amino acid sequence of SEQ ID NO: 6, and referred to Bioneer for gene synthesis. The rIL-10 gene was amplified by conducting PCR using the rat IL-10 gene introduced into pGEM-T easy as a template, and then the resultant was treated with NheI and SalI to prepare a 0.5 Kb DNA fragment. In addition, PGEM-T-CMV was treated with NheI and SalI to prepare a 3.7 Kb DNA fragment. The two DNA fragments were ligated to prepare pGEM-T-CMV-rIL-10. The primer sequences used for rIL-10 amplification are as follows:
F-rIL-10 (SEQ ID NO: 23): 5'-CCGCTAGCGCCACCATGCCT-3'
R-rIL-10 (SEQ ID NO: 24): 5'-GACGTCGACGCCATCGATGGCTTAATTAATCAATTCTTC-3'.

As for the SV40pA, the gene was amplified by conducting PCR using pCI as a template and then treated with NotI and SalI to prepare a 222 bp DNA fragment. In addition, the pGEM-T-CMV-rIL-10 was treated with ClaI and SalI to prepare a 4.2 Kb DNA fragment. The two DNA fragments were ligated to construct pGEM-T-CMV-rIL-10-SV40pA. The primer sequences used for SV40pA amplification are as follows:
F-SV40pA (SEQ ID NO: 17): 5'-CCATCGATCAGACATGATAAGATACATTGATGAG-3'
R-SV40pA (SEQ ID NO: 18): 5'-GACGTCGACGCGGCCGCTACCACATTTGTAGAGGTTTTACTTG-3'.

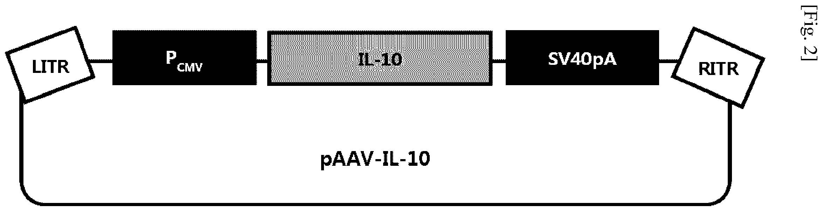

pGEM-T-CMV-rIL-10-SV40pA was treated with EagI to prepare a 1.6 Kb DNA fragment. In addition, pAAV-MCS-Kan^{r} was treated with NotI and BamHI to prepare DNA fragments. Thereafter, the two DNA fragments were ligated to construct pssAAV-CMV-rIL-10-SV40pA (hereinafter abbreviated as "pAAV-IL-10"). The schematic diagram of the pAAV-IL-10 plasmid is shown in Fig. 2.

### Example 1.3. Construction of pAAV-GDNF plasmid

As for the human GDNF gene, the gene represented by the SEQ ID NO: 13 was designed by codon-optimization to be suitable for humans based on human GDNF (NCBI NM_199231.2) represented by the amino acid sequence of SEQ ID NO: 11, and referred to Bioneer for gene synthesis. The hGDNF gene introduced into the pGEM-B1 plasmid was treated with NheI and PacI to prepare a DNA fragment of about 0.6 kb. The pGEM-T-CMV-rIL-10-SV40pA plasmid was treated with NheI and PacI to prepare a 2.8 kb fragment in which the rIL-10 gene was removed. The two DNA fragments were ligated to construct the pGEM-T-CMV-hGDNF-SV40pA plasmid.

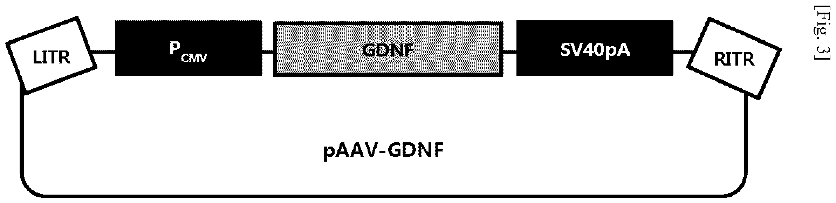

Then, the completed pGEM-T-CMV-hGDNF-SV40pA plasmid was treated with EagI to prepare a 1.5 kb DNA fragment. In addition, pAAV-MCS-Kan^{r} was treated with NotI and BamHI to prepare a 1.8 kb DNA fragment. The two DNA fragments were ligated to construct pssAAV-CMV-hGDNF-SV40pA-Kan^{r} (hereinafter abbreviated as "pAAV-GDNF"). The schematic diagram of the pAAV-GDNF plasmid is shown in Fig. 3.

### Example 1.4. Construction of pAAV-GDNF-IL-10 plasmid

The CAG promoter (cytomegalovirus enhancer, chicken β-actin promoter, and rabbit β-globin poly A signal) was subjected to PCR amplification using pAxCAwtit2 contained in the Adenovirus dual expression kit (Takara), and treated with ApaI and XbaI to improve expression, thereby removing about 80% of the chicken β-actin region in the CAG promoter to produce a short CAG (sCAG) promoter (Fagoe, Gene Ther, 2014). As for the human IL-10 gene, the gene encoding the SEQ ID NO: 14 was designed by codon-optimization of the gene encoding SEQ ID NO: 9 to be suitable for humans, and referred to Bioneer for gene synthesis. Next, DNA fragment for bovine growth hormone (bGH) poly A was obtained by PCR amplification. The pVAX1/sCAG-hIL-10-bGHpA was constructed using pVAX1 (Invitrogen) to contain the promoter and poly A and human IL-10 genes.

Next, pVAX1/CMV-hGDNF-SV40pA was prepared by the same method as in the Example 1.1., using the human GDNF gene of the Example 1.3. Thereafter, the SV40pA-hGDNF-CMV gene cassette was amplified by conducting PCR using pVAX1/CMV-hGDNF-SV40pA as a template, and a 1.5 kb DNA fragment was prepared. The primer sequences used for the gene cassette amplification are as follows:
SV40-CMV-sCAG-bGHpA-Infu-F (SEQ ID NO: 27):
5'-CCTGCGGCCGGTCGACTACCACATTTGTAGAGGTTTTACTTGC-3'
SV40-CMV-sCAG-bGHpA-Infu-R (SEQ ID NO: 28):
5'-AATAATCAATGTCGACTCGAGGAGCTTGGCCCATT-3'

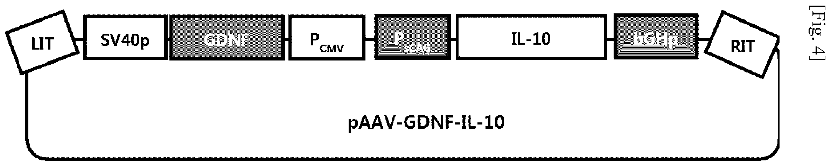

Next, pVAX1/sCAG-hIL-10-bGHpA was treated with SalI to prepare a DNA fragment of about 3.9 kb, and the 1.5 kb DNA fragment described above was inserted into the 3.9 kb DNA fragment using an In-Fusion HD Cloning Kit (Clontech) to construct pVAX1/SV40pA-hGDNF-CMV-sCAG-hIL-10-bGHpA (hereinafter abbreviated as "pAAV-GDNF-IL-10"). The pAAV-GDNF-IL-10 plasmid is schematically shown in Fig. 4.

### Experimental Example 1. Confirmation of expression of pAAV-GAD65, pAAV-IL-10, pAAV-GDNF and pAAV-GDNF-IL-10 plasmids

The pAAV-GAD65, pAAV-IL-10, pAAV-GDNF, or pAAV-GDNF-IL-10 plasmids prepared in the Examples 1.1. to 1.4 were respectively transfected into human embryonic kidney cell line 293T cells using jetPRIME (Polyplus). The transfected cells were cultured in a 37°C incubator for 48 hours. Thereafter, the cell culture medium or cultured cells were harvested. The cells were dissolved with a solvent, and the prepared samples were treated with each of the antibodies to GAD65 (Merck Millipore), IL-10 (Santa Cruz), and GDNF (R&D systems), and subjected to Western blotting.

Specifically, in the case of pAAV-GAD65, the human embryonic kidney cell line 293T cells were treated with 2 µg of pAAV-GAD65 plasmid and cultured for 48 hours. Thereafter, the cultured cells were dissolved and the expression of GAD65 in the cells was confirmed through Western blotting.

In the case of pAAV-IL-10 and pAAV-GDNF plasmids, the human embryonic kidney cell line 293T cells were treated with 1 µg of pAAV-IL-10 or pAAV-GDNF plasmid, and cultured for 48 hours. Thereafter, the culture medium was harvested and the expression of IL-10 or GDNF in the medium was confirmed through Western blotting.

In the case of the pAAV-GDNF-IL-10 plasmid, the human embryonic kidney cell line 293T cells were treated with 1 µg of pAAV-GDNF-IL-10 plasmid and cultured for 48 hours. Thereafter, the cultured cells were dissolved, and the expression of intracellular IL-10 and GDNF was confirmed through Western blotting.

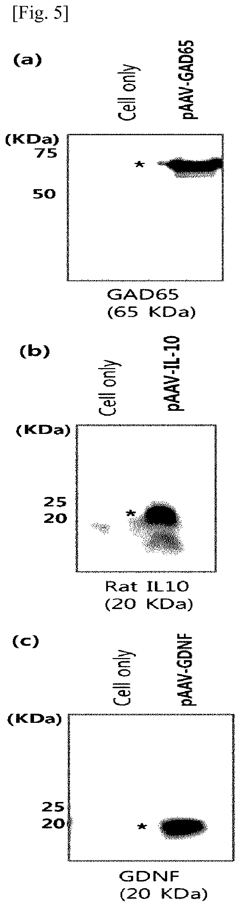

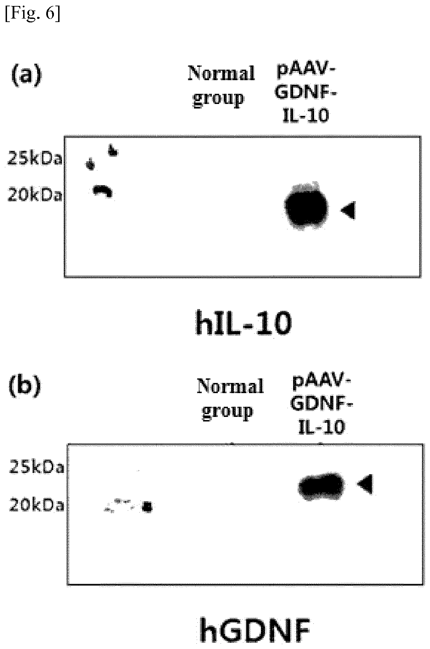

As a result, it was confirmed that the transfected pAAV-GAD65, pAAV-IL-10, pAAV-GDNF or pAAV-GDNF-IL-10 plasmid was expressed (Figs. 5 and 6).

### Example 2. Preparation of recombinant adeno-associated virus

The AAV-IL-10 virus used in the experiment was produced and purified by UNC vector core. The production method is as follows. The pVax-rIL-10, pHelper and pRC5 were transfected into human embryonic kidney cell line 293T cells. Thereafter, the resultant was subject to purification by column chromatography to secure AAV5-IL-10 virus. The titer of the produced virus was measured using qPCR.

The AAV-GAD65 and AAV-GDNF viruses were produced and purified by KRcrogen. The production method is as follows. The AAV-transgenes (pAAV-GAD65 and pAAV-GDNF plasmids) were respectively transfected into the human embryonic kidney cell line 293T cells using the calcium phosphate method along with pHelper and pRC. In the case of GAD65, pRC5 introduced with the capsid gene of serotype 5 was used. In the case of GDNF, pRC1 introduced with the capsid gene of AAV serotype 1 was used. The transfected cells were cultured in a 37°C incubator and the cells were harvested after 48 hours.

Thereafter, only the bands containing viruses were isolated and purified through the ultrahigh speed centrifugation method according to the cesium concentration gradient, to secure AAV5-GAD65, and AAV1-GDNF viruses. The titers of the produced viruses were measured using qPCR.

The AAV-GDNF-IL-10 virus was produced and purified by Cdmogen. The production method is as follows. The AAV-transgene (pAAV-GDNF-IL-10 plasmid) was transfected into the human embryonic kidney cell line 293T cells using the calcium phosphate method along with pHelper and pRC5. The transfected cells were cultured in a 37°C incubator and the cells were harvested after 48 hours.

Thereafter, only the bands containing viruses were isolated and purified through ultrahigh speed centrifugation according to the cesium concentration gradient, to secure AAV5-GDNF-IL-10 virus. The titer of the produced virus was measured using qPCR.

The AAV-GAD65-modi virus was produced and purified by Cdmogen. The production method is as follows. The AAV-transgene (pAAV-hGAD65-modi plasmid) was transfected into human embryonic kidney cell line 293T cells using the calcium phosphate method along with pHelper and pRC5. The transfected cells were cultured in a 37°C incubator and the cells were harvested after 48 hours.

Thereafter, only the bands containing viruses were isolated and purified by ultrahigh-speed centrifugation according to the cesium concentration gradient to secure the AAV5-GAD65-modi virus. The titer of the produced virus was measured using qPCR.

### Experimental Example 2. Property analysis of recombinant adeno-associated virus

In order to examine the protein expression of recombinant adeno-associated virus delivered into a cell, human embryonic kidney cell line 293T or HeLa cells were treated with the AAV-GAD65, AAV-GAD65-modi, AAV-IL-10 or AAV-GDNF virus obtained above, and protein expression was examined by Western blotting. Specifically, 293T or HeLa cells were seeded at 5 × 10⁵ cells/well in a 6-well plate. And on the next day, the cells were respectively treated with 3 types of viruses at 10,000 vg/well, and then cultured in a 37°C incubator. After 48 hours, the cells were harvested and were dissolved with a solvent and the culture medium was concentrated using the amicon (Merck Millipore). Then, the prepared samples were respectively treated with the antibodies to GAD65 (Cell signaling), IL-10 (Santa Cruz) and GDNF (R&D systems), and subjected to Western blotting.

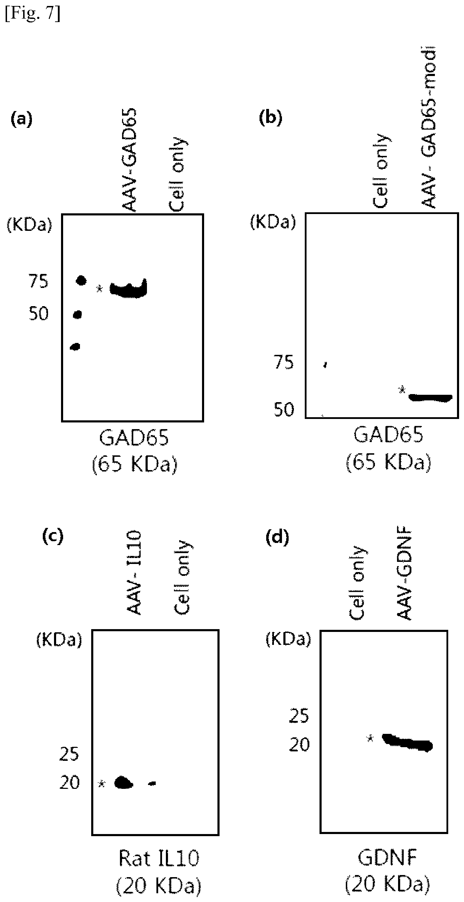

As a result, it was confirmed that each target protein was expressed in the cell lysate of human embryonic kidney cell line 293T or HeLa cell line treated with AAV-GAD65, AAV-GAD65-modi, AAV-IL-10 or AAV-GDNF virus (Fig. 7). Therefore, it was confirmed that there was no abnormality in the structures and properties of the recombinant adeno-associated viruses used in the experiment.

Also, in order to confirm that GABA is produced by AAV-GAD65 or AAV-GAD65-modi virus, the culture medium of the cells treated with the AAV-GAD65 or AAV-GAD65-modi virus was harvested and subjected to GABA ELISA (LDN) analysis. For each experimental group, two identical samples were prepared separately to conduct the analysis, and the bar graph shows the value for each sample.

As a result, it was confirmed that GABA was secreted to the culture medium by GAD65 introduced into cells by AAV-GAD65 or AAV-GAD65-modi virus (Fig. 8).

### Experimental Example 3. Comparison of analgesic efficacies between individual administration of AAV-GAD65, AAV-IL-10 or AAV-GDNF virus and co-administration of AAV-GAD65 and AAV-GDNF, or AAV-IL-10 and AAV-GDNF viruses

### Experimental Example 3.1. Preparation of administration sample

The viruses prepared in Example 2 were used for the test. 30 minutes prior to the animal administration experiment, the reagents stored at -80°C were thawed at room temperature and prepared by mixing by a vortexer. AAV-GAD65, AAV-IL-10, AAV-GDNF, and AAV-GFP viruses were diluted in PBS to obtain the titers shown in Table 1. The AAV-GFP virus was administered in the same amount as other recombinant adeno-associated viruses. The GFP is a protein having no analgesic efficacy. The viruses required were mixed according to the contents indicated in Table 1, and administered in an amount of 9.0 × 10⁸ vg/5 µl per animal (vg: virus genome).

**[Table 1]**

| Samples | Virus types and contents | | | |
|---|---|---|---|---|
| | AAV-GAD65 | AAV-IL-10 | AAV-GDNF | AAV-GFP |
| AAV-GFP | - | - | - | 9.0 × 10⁸ vg/5 µl |
| AAV-GAD65 | 9.0 × 10⁸ | - | - | - |
| | vg/5 µl | | | |
| AAV-IL-10 | - | 9.0 × 10⁸ vg/5 µl | - | - |
| AAV-GDNF | - | - | 9.0 × 10⁸ vg/5 µl | - |
| AAV-GAD65 + AAV-GDNF | 4.5 × 10⁸ vg/5 µl | - | 4.5 × 10⁸ vg/5 µl | - |
| AAV-IL-10 + AAV-GDNF | - | 4.5 × 10⁸ vg/2.5 µl | 4.5 × 10⁸ vg/2.5 µl | - |

### Experimental Example 3.2. Construction of neuropathic pain-induced rats and administration of samples

150 to 200 g male SD-rats were subjected to inhalation anesthesia. And then the upper part of the calf was incised and both ends of the common peroneal nerve and tibial nerve were tied and knots were made at the interval of 0.5 to 1 cm by 7-0 suture. The regions between the knots of the two nerve bundles were cut with a scissor and the incision site was sutured. Thereafter, the rats were recovered to be awakened from the anesthesia and returned to the cage. Two weeks later, the *von Frey* filament test was conducted to examine pain induction, and then the samples prepared in Example 2.1 were respectively administered (Decosterd, Pain, 2000).

The samples were administered by the transforaminal epidural injection method at a location adjacent to the dorsal root ganglion (DRG). The pain-induced rat was subjected to inhalation anesthesia, and the vertebrae were exposed by linearly incising the back of the rat at the levels of lumbar spines L3 to L5. At the side of the exposed space, the L4 transverse process, one of the spinal projections, was made visible. The rat was laid down sideways such that its lateral side is visible from above and the L4 intervertebral foramen was visible.

Thereafter, a needle attached to the catheter was inserted into the prepared sample, and a Hamilton syringe was connected to the opposite end of the catheter and pulled to the marking line of 5 µl to inject the sample into the catheter. The Hamilton syringe was removed from the catheter and then the catheter was secured by holding the point 1 cm away from the tip of the needle using Halsted-Mosquito. Then, while holding and pulling the L4 spine upward with tweezers, the tip of the needle secured by Halstead Mosquito was taken around the L4 intervertebral foramen with the other hand. The tip of the needle was inserted into the bent region inside the intervertebral foramen whose space was secured. Then, the needle which was being held was released.

After confirming that the needle was fixed, a 1 ml syringe was connected to the catheter connected to the opposite side of the needle. By gently pressing the piston of the syringe, the sample was slowly injected around the dorsal root ganglion of the rat. Thereafter, the incision site was sutured. 4 weeks after the sample administration, pain responses were observed using *von Frey* filament test.

### Experimental Example 3.3. Pain observation using von Frey filament test

Pain was observed using the *von Frey* filament test. The method is to calculate the threshold value according to a predetermined pattern of the pain response with a total of eight filaments of 0.4, 0.6, 1, 2, 4, 6, 8 and 15 g.

Pain generating regions were searched by changing the position from the beginning portion of the outermost toe to the heel of the sole where pain was generated. Since the rats suddenly took off the soles and shrank or licked their soles with their mouths when pain was generated, the pain generating regions could be found. If there were reactions three times or more when the surrounding area was pricked five times with the filament of each step, it was regarded as a pain response. And the test was proceeded by replacing the filament with that of the next step. In this way, the pattern of each step was recorded.

The pain patterns were recorded based on the pattern table established by S.R. Chaplan, and the threshold values were calculated using the pain patterns (Chaplan, J Neurosci Methods, 1994). As for the behavioral analysis, the animal groups were blinded at a specified time and at least 3 researchers observed, and the results of recorded patterns were statistically processed, to analyze the pain results.

As a result, it was found that the pain-alleviating effect was higher when AAV-GAD65 and AAV-GDNF, or AAV-IL-10 and AAV-GDNF viruses were combined and co-administered as compared to individual administration of AAV-GAD65, AAV-IL-10 or AAV-GDNF virus (Fig. 9).

### Experimental Example 4. Comparison of analgesic efficacies between individual administration of AAV-GAD65, AAV-IL-10 or AAV-GDNF virus and co-administration of AAV-GAD65, AAV-IL-10, and AAV-GDNF viruses

The viruses prepared in Example 2 were used for the test. 30 minutes prior to the animal administration experiment, the reagents stored at -80°C were thawed at room temperature and prepared by mixing by a vortexer. AAV-GAD65, AAV-IL-10, AAV-GDNF, and AAV-GFP viruses were diluted in PBS to obtain the titers shown in Table 2. The AAV-GFP virus was administered in the same amount as other recombinant adeno-associated viruses. The GFP is a protein whose analgesic efficacy has not been reported. The viruses were mixed according to the contents shown in Table 2, and administered in an amount of 9.0 × 10⁸ vg/5 µl per animal.

**[Table 2]**

| Samples | Virus types and contents | | | |
|---|---|---|---|---|
| | AAV-GAD65 | AAV-IL-10 | AAV-GDNF | AAV-GFP |
| AAV-GFP | - | - | - | 9.0 × 10⁸ vg/5 µl |
| AAV-GAD65 | 9.0 × 10⁸ vg/5 µl | - | - | - |
| AAV-IL-10 | - | 9.0 × 10⁸ vg/5 µl | - | - |
| AAV-GDNF | - | - | 9.0 × 10⁸ vg/5 µl | - |
| AAV-GAD65 + AAV-IL-10 + AAV-GDNF | 3.0 × 10⁸ vg/5 µl | 3.0 × 10⁸ vg/5 µl | 3.0 × 10⁸ vg/5 µl | - |

To the pain animal model produced by the same method as in Experimental Example 3.2., samples prepared according to the virus contents shown in Table 2 were administered. Thereafter, the *von Frey* filament test was conducted by the same method as in Experimental Example 3.3. to observe the pain responses.

As a result, it was found that the pain-alleviating effect was higher when all of the AAV-GAD65, AAV-IL-10 and AAV-GDNF viruses were co-administered as compared to individual administration of AAV-GAD65, AAV-IL-10 or AAV-GDNF virus (Fig. 10).

### Experimental Example 5. Comparison of analgesic efficacies between co-administration of AAV-GAD65 and AAV-GDNF, or AAV-IL-10 and AAV-GDNF viruses and co-administration of AAV-GAD65, AAV-IL-10, and AAV-GDNF viruses

The adeno-associated viruses prepared in Example 2 were used for the test. 30 minutes prior to the animal administration experiment, the reagents stored at -80°C were thawed at room temperature and prepared by mixing by a vortexer. AAV-GAD65, AAV-IL-10, AAV-GDNF, and AAV-GFP viruses were diluted in PBS to obtain the titers shown in Table 3. The AAV-GFP was administered in the same amount as other recombinant adeno-associated viruses. The GFP is a protein which has no analgesic efficacy. The viruses required were mixed according to the contents shown in Table 3, and administered in an amount of 9.0 × 10⁸ vg/5 µl per animal.

**[Table 3]**

| Samples | Virus types and contents | | | |
|---|---|---|---|---|
| | AAV-GAD65 | AAV-IL-10 | AAV-GDNF | AAV-GFP |
| AAV-GFP | - | - | - | 9.0 × 10⁸ vg/5 µl |
| AAV-GAD65 + AAV-GDNF | 4.5 × 10⁸ vg/5 µl | - | 4.5 × 10⁸ vg/5 µl | - |
| AAV-IL-10 + AAV-GDNF | - | 4.5 × 10⁸ vg/5 µl | 4.5 × 10⁸ vg/5 µl | - |
| AAV-GAD65 + AAV-IL-10 + AAV-GDNF | 3.0 × 10⁸ vg/5 µl | 3.0 × 10⁸ vg/5 µl | 3.0 × 10⁸ vg/5 µl | - |

To the pain animal model produced by the same method as in Experimental Example 3.2., samples prepared according to the virus contents shown in Table 3 were administered. Thereafter, the *von Frey* filament test was conducted by the same method as in Experimental Example 3.3. to observe the pain responses.

As a result, it was found that the pain-alleviating effect was higher when all of the AAV-GAD65, AAV-IL-10 and AAV-GDNF viruses were co-administered as compared to co-administration of AAV-GAD65 and AAV-GDNF, or AAV-IL-10 and AAV-GDNF viruses (Fig. 11).

### Experimental Example 6. Comparison of analgesic efficacies between individual administration of pAAV-GAD65, pAAV-IL-10, or pAAV-GDNF plasmid and co-administration of pAAV-GAD65 and pAAV-GDNF, or pAAV-IL-10 and pAAV-GDNF plasmids

### Experimental Example 6.1. Preparation of administration samples

The plasmids prepared in Example 1 were used for the test. 30 minutes prior to the animal administration experiment, the reagents stored at -80°C were thawed at room temperature and prepared by mixing by a vortexer. The pAAV-GAD65, pAAV-IL-10, pAAV-GDNF, and pVAX1 plasmids were diluted in the Tris-EDTA buffer to obtain the concentrations shown in Table 4. The pVAX1 plasmid was administered in the same amount as the other plasmids. The pVAX1 plasmid has not been reported to have an analgesic efficacy. The required plasmids were mixed according to the contents shown in Table 4, and administered in an amount of 30 µg/50 µl per animal.

**[Table 4]**

| Samples | Plasma types and contents | | | |
|---|---|---|---|---|
| | pAAV-GAD65 | pAAV-IL-10 | pAAV-GDNF | pVAX1 |
| pVAX1 | - | - | - | 30 µg/50 µl |
| pAAV-GAD65 | 30 µg/50 µl | - | - | - |
| pAAV-IL-10 | - | 30 µg/50 µl | - | - |
| pAAV-GDNF | - | - | 30 µg/50 µl | - |
| pAAV-GAD65 + pAAV-GDNF | 15 µg/50 µl | - | 15 µg/50 µl | - |
| pAAV-IL-10 + pAAV-GDNF | - | 15 µg/50 µl | 15 µg/50 µl | - |

### Experimental Example 6.2. Construction of neuropathic pain animal model and sample administration

150 to 200 g male SD-rats were subjected to inhalation anesthesia. And then the upper part of the calf was incised and both ends of the common peroneal nerve and tibial nerve were tied and knots were made at the interval of 0.5 to 1 cm by 7-0 suture. The regions between the knots of the two nerve bundles were cut with a scissor and the incision site was sutured. Thereafter, the rats were recovered to be awakened from the anesthesia and returned to the cage. Two weeks later, the *von Frey* filament test was conducted to examine pain induction, and then the samples prepared in Example 6.1 were administered (Decosterd, Pain, 2000).

The samples were administered by the intrathecal injection method. The pain-induced rat was subjected to inhalation anesthesia, and the spinous process was exposed by linearly incising the back of the rat at the region of lumbar spines L5. A 50 ml tube was placed under the rat to widen the space between L5 and L6, and a needle of 27 G × 13 mm size was inserted. Thereafter, a 1 mL syringe filled with 50 µl of the sample was connected to the needle. The sample was slowly injected by slightly pressing the piston of the syringe. Thereafter, the incision was sutured and this step was finished. One day after the substance administration, the pain responses were observed using the *von Frey* filament test.

### Experimental Example 6.3. Pain observation using von Frey filament test

The *von Frey* filament test was carried out by the same method as in Experimental Example 3.3. As a result, it was found that the pain-alleviating effect was higher when pAAV-GAD65 and pAAV-GDNF, or pAAV-IL-10 and pAAV-GDNF plasmids were co-administered as compared to individual administration of pAAV-GAD65, pAAV-IL-10 or pAAV-GDNF plasmid (Fig. 12).

### Experimental Example 7. Comparison of analgesic efficacies between individual administration of pAAV-GAD65, pAAV-IL-10, or pAAV-GDNF plasmid and co-administration of pAAV-GAD65, pAAV-IL-10 and pAAV-GDNF plasmids

The plasmids prepared in Example 1 were used for the test. 30 minutes prior to the animal administration experiment, the reagents stored at -80°C were thawed at room temperature and prepared by mixing by a vortexer. The pAAV-GAD65, pAAV-IL-10, pAAV-GDNF, and pVAX1 plasmids were diluted in the Tris-EDTA buffer to obtain the concentrations shown in Table 5. The pVAX1 plasmid was administered in the same amount as the other plasmids. The pVAX1 plasmid has not been reported to have an analgesic efficacy. The required plasmids were mixed according to the contents shown in Table 5, and administered in an amount of 30 µg/50 µl per animal.

**[Table 5]**

| Samples | Plasma types and contents | | | |
|---|---|---|---|---|
| | pAAV-GAD65 | pAAV-IL-10 | pAAV-GDNF | pVAX1 |
| pAAV-VAX1 | - | - | - | 30 µg/50 µl |
| pAAV-GAD65 | 30 µg/50 µl | - | - | - |
| pAAV-IL-10 | - | 30 µg/50 µl | - | - |
| pAAV-GDNF | - | - | 30 µg/50 µl | - |
| pAAV-GAD65 + pAAV-IL-10 + pAAV-GDNF | 10 µg/50 µl | 10 µg/50 µl | 10 µg/50 µl | - |

To the pain animal model produced by the same method as in Experimental Example 6.2., samples prepared according to the virus contents shown in Table 5 were administered. Thereafter, the *von Frey* filament test was conducted by the same method as in Experimental Example 3.3. to observe the pain responses.

As a result, it was found that the pain-alleviating effect was higher when all of the pAAV-GAD65, pAAV5-IL-10, and pAAV5-GDNF plasmids were co-administered as compared to individual administration of pAAV-GAD65, pAAV-IL-10 or pAAV-GDNF plasmid (Fig. 13).

### Experimental Example 8. Comparison of the analgesic efficacies between co-administration of pAAV-GAD65 and pAAV-GDNF, or pAAV-IL-10 and pAAV-GDNF plasmids and co-administration of pAAV-GAD65, pAAV-IL-10 and pAAV-GDNF plasmids

The plasmids prepared in Example 1 were used for the test. 30 minutes prior to the animal administration experiment, the reagents stored at -80°C were thawed at room temperature and prepared by mixing by a vortexer. The pAAV-GAD65, pAAV-IL-10, pAAV-GDNF, and pVAX1 plasmids were diluted in the Tris-EDTA buffer to obtain the concentrations shown in Table 6. The pVAX1 plasmid was administered in the same concentration and amount as the other plasmids. The pVAX1 plasmid has not been reported to have an analgesic efficacy. The required plasmids were mixed according to the contents shown in Table 6, and administered in an amount of 30 µg/50 µl per animal.

**[Table 6]**

| Samples | Plasma types and contents | | | |
|---|---|---|---|---|
| | pAAV-GAD65 | pAAV-IL-10 | pAAV-GDNF | pVAX1 |
| pVAX1 | - | - | - | 30 µg/50 µl |
| pAAV-GAD65 + pAAV-GDNF | 15 µg/50 µl | - | 15 µg/50 µl | - |
| pAAV-IL-10 + pAAV-GDNF | - | 15 µg/50 µl | 15 µg/50 µl | - |
| pAAV-GAD65 + pAAV-IL-10 + pAAV-GDNF | 10 µg/50 µl | 10 µg/50 µl | 10 µg/50 µl | - |

To the pain animal model produced by the same method as in Experimental Example 6.2., samples prepared according to the plasma contents shown in Table 6 were administered. Thereafter, the *von Frey* filament test was conducted by the same method as in Experimental Example 6.3. to observe the pain responses.

As a result, it was found that the pain-alleviating effect was higher when all of the pAAV-GAD65, pAAV-IL-10 and pAAV-GDNF plasmids were co-administered as compared to co-administration of pAAV-GAD65 and pAAV-GDNF or pAAV-IL-10 and pAAV-GDNF plasmids (Fig. 14).

### Experimental Example 9. Comparison of analgesic efficacies of co-administration of AAV-GAD65-modi and AAV-GDNF-IL-10 viruses and co-administration of AAV-GAD65, AAV-IL-10 and AAV-GDNF viruses

The adeno-associated viruses prepared in Example 2 were used for the test. 30 minutes prior to the animal administration experiment, the reagents stored at -80°C were thawed at room temperature and prepared by mixing by a vortexer. AAV-GAD65-modi, AAV-GDNF-IL-10, AAV-GAD65, AAV-IL-10 and AAV-GDNF viruses were diluted in PBS to obtain the titers shown in Table 7. The viruses required were mixed according to the contents shown in Table 7, and administered in an amount of 1.0 × 10⁹ vg/5 µl or 1.5 × 10⁹ vg/5 µl per animal.

**[Table 7]**

| Samples | Virus types and contents | | | | |
|---|---|---|---|---|---|
| | AAV-GAD65-modi | AAV-GDNF-IL-10 | AAV-GAD65 | AAV-IL-10 | AAV-GDNF |
| Control | - | - | - | - | - |
| AAV-GAD65-modi + AAV-GDNF-IL-10 | 5.0 × 10⁸ vg/5 µl | 5.0 × 10⁸ vg/5 µl | - | - | - |
| AAV-GAD65 + AAV-IL-10 + AAV-GDNF | - | - | 5.0 × 10⁸ vg/5 µl | 5.0 × 10⁸ vg/5 µl | 5.0 × 10⁸ vg/5 µl |

To the pain animal model produced by the same method as in Experimental Example 3.2., samples prepared according to the virus contents shown in Table 7 were administered. Thereafter, the *von Frey* filament test was conducted by the same method as in Experimental Example 3.3. to observe the pain responses.

As a result, it was found that the pain-alleviating effect was higher when AAV-GAD65-modi and AAV-GDNF-IL-10 viruses were co-administered as compared to co-administration of all of AAV-GAD65, AAV-IL-10 and AAV-GDNF viruses (Fig. 15).
## Claims

1. A pharmaceutical composition for use in alleviating or treating pain comprising two or more selected from the group consisting of a gene encoding glutamate decarboxylase (GAD), a gene encoding interleukin-10 (IL-10), and a gene encoding a glial cell-derived neurotrophic factor (GDNF).

2. The pharmaceutical composition for use according to claim 1, wherein the gene is in a form of being operably contained in a vector.

3. The pharmaceutical composition for use according to claim 2, wherein the vector is at least one viral vector selected from the group consisting of adenovirus, adeno-associated virus, herpes simplex virus, lentivirus, retrovirus, and poxvirus; or wherein the vector is at least one non-viral vector selected from the group consisting of a plasmid, a liposome, a cationic polymer, a micelle, an emulsion, and solid lipid nanoparticles.

4. The pharmaceutical composition for use according to claim 1, wherein the GAD is GAD65 or GAD67.

5. The pharmaceutical composition for use according to claim 1, wherein the gene encoding GAD is the nucleotide sequence encoding the amino acid sequence represented by SEQ ID NO: 1, SEQ ID NO: 4, SEQ ID NO: 32, SEQ ID NO: 34 or SEQ ID NO: 36.

6. The pharmaceutical composition for use according to claim 1, wherein the gene encoding GAD is the nucleotide sequence represented by SEQ ID NO: 2, SEQ ID NO: 3, SEQ ID NO: 5, SEQ ID NO: 33, SEQ ID NO: 35 or SEQ ID NO: 37.

7. The pharmaceutical composition for use according to claim 1, wherein the gene encoding IL-10 is the nucleotide sequence encoding the amino acid sequence represented by SEQ ID NO: 6, SEQ ID NO: 9, SEQ ID NO: 38, SEQ ID NO: 40 or SEQ ID NO: 42.

8. The pharmaceutical composition for use according to claim 1, wherein the gene encoding IL-10 is the nucleotide sequence represented by SEQ ID NO: 7, SEQ ID NO: 8, SEQ ID NO: 10, SEQ ID NO: 14, SEQ ID NO: 39, SEQ ID NO: 41 or SEQ ID NO: 43.

9. The pharmaceutical composition for use according to claim 1, wherein the gene encoding GDNF is the nucleotide sequence encoding the amino acid sequence represented by SEQ ID NO: 11, SEQ ID NO: 44, SEQ ID NO: 46, or SEQ ID NO: 48.

10. The pharmaceutical composition for use according to claim 1, wherein the gene encoding GDNF is the nucleotide sequence represented by SEQ ID NO: 12, SEQ ID NO: 13, SEQ ID NO: 45, SEQ ID NO: 47 or SEQ ID NO: 49.

11. The pharmaceutical composition for use according to claim 1, wherein the pain is nociceptive pain, psychogenic pain, inflammatory pain, pathological pain, neuropathic pain, cancer pain, postoperative pain, trigeminal neuralgia pain, idiopathic pain, diabetic neuropathic pain, or migraine.

12. The pharmaceutical composition for use according to claim 1, wherein the pharmaceutical composition further comprises a physiologically acceptable carrier; or wherein the pharmaceutical composition is an injection.

13. The pharmaceutical composition for use according to claim 1, wherein the pharmaceutical composition is formulated for administration via epidural injection or intrathecal injection.

## Patentansprüche

1. Eine pharmazeutische Zusammensetzung zur Verwendung bei der Linderung oder Behandlung von Schmerz, umfassend zwei oder mehr ausgewählt aus der Gruppe bestehend aus einem Gen, das die Glutamat Decarboxylase (GAD) kodiert, einem Gen, das Interleukin-10 (IL-10) kodiert und einem Gen, das einen Gliazell-abgeleiteten Neurotrophen Faktor (GDNF) kodiert.

2. Die pharmazeutische Zusammensetzung zur Verwendung nach Anspruch 1, wobei das Gen in einer Form ist, dass es operativ in einem Vektor enthalten ist.

3. Die pharmazeutische Zusammensetzung zur Verwendung nach Anspruch 2, wobei der Vektor mindestens ein viraler Vektor ist ausgewählt aus der Gruppe bestehend aus Adenovirus, Adeno-assoziiertem Virus, Herpes Simplex Virus, Lentivirus, Retrovirus und Pockenvirus; oder wobei der Vektor mindestens ein nicht-viraler Vektor ist ausgewählt aus der Gruppe bestehend aus einem Plasmid, einem Liposom, einem kationischen Polymer, einer Micelle, einer Emulsion und festen Lipid-Nanopartikeln.

4. Die pharmazeutische Zusammensetzung zur Verwendung nach Anspruch 1, wobei GAD GAD65 oder GAD67 ist.

5. Die pharmazeutische Zusammensetzung zur Verwendung nach Anspruch 1, wobei das Gen, das GAD kodiert, die Nukleotidsequenz ist, die die Aminosäuresequenz kodiert, die repräsentiert ist durch SEQ ID NO: 1, SEQ ID NO: 4, SEQ ID NO: 32, SEQ ID NO: 34 oder SEQ ID NO: 36.

6. Die pharmazeutische Zusammensetzung zur Verwendung nach Anspruch 1, wobei das Gen, das GAD kodiert, die Nukleotidsequenz ist, die repräsentiert ist durch SEQ ID NO: 2, SEQ ID NO: 3, SEQ ID NO: 5, SEQ ID NO: 33, SEQ ID NO: 35 oder SEQ ID NO: 37.

7. Die pharmazeutische Zusammensetzung zur Verwendung nach Anspruch 1, wobei das Gen, das IL-10 kodiert, die Nukleotidsequenz ist, die die Aminosäuresequenz kodiert, die repräsentiert ist durch SEQ ID NO: 6, SEQ ID NO: 9, SEQ ID NO: 38, SEQ ID NO: 40 oder SEQ ID NO: 42.

8. Die pharmazeutische Zusammensetzung zur Verwendung nach Anspruch 1, wobei das Gen, das IL-10 kodiert, die Nukleotidsequenz ist, die repräsentiert ist durch SEQ ID NO: 7, SEQ ID NO: 8, SEQ ID NO: 10, SEQ ID NO: 14, SEQ ID NO: 39, SEQ ID NO: 41 oder SEQ ID NO: 43.

9. Die pharmazeutische Zusammensetzung zur Verwendung nach Anspruch 1, wobei das Gen, das GDNF kodiert, die Nukleotidsequenz ist, die die Aminosäuresequenz kodiert, die repräsentiert ist durch SEQ ID NO: 11, SEQ ID NO: 44, SEQ ID NO: 46 oder SEQ ID NO: 48.

10. Die pharmazeutische Zusammensetzung zur Verwendung nach Anspruch 1, wobei das Gen, das GDNF kodiert, die Nukleotidsequenz ist, die repräsentiert ist durch SEQ ID NO: 12, SEQ ID NO: 13, SEQ ID NO: 45, SEQ ID NO: 47 oder SEQ ID NO: 49.

11. Die pharmazeutische Zusammensetzung zur Verwendung nach Anspruch 1, wobei der Schmerz nozizeptiver Schmerz, psychogener Schmerz, entzündlicher Schmerz, pathologischer Schmerz, neuropathischer Schmerz, Krebsschmerz, postoperativer Schmerz, trigeminaler neuralgischer Schmerz, idiopathischer Schmerz, diabetisch neuropathischer Schmerz oder Migräne ist.

12. Die pharmazeutische Zusammensetzung zur Verwendung nach Anspruch 1, wobei die pharmazeutische Zusammensetzung ferner einen physiologisch annehmbaren Träger umfasst oder wobei die pharmazeutische Zusammensetzung eine Injektion ist.

13. Die pharmazeutische Zusammensetzung zur Verwendung nach Anspruch 1, wobei die pharmazeutische Zusammensetzung für die Verabreichung via epiduraler Injektion oder intrathekaler Injektion formuliert ist.

## Revendications

1. Composition pharmaceutique destinée à être utilisée pour soulager ou traiter la douleur, comprenant au moins deux choisis dans le groupe consistant en un gène codant pour la glutamate décarboxylase (GAD), un gène codant pour l'interleukine-10 (IL-10) et un gène codant pour un facteur neurotrophique dérivé des cellules gliales (GDNF) .

2. Composition pharmaceutique pour utilisation selon la revendication 1, dans laquelle le gène est sous la forme d'être contenu de manière opérationnelle dans un vecteur.

3. Composition pharmaceutique pour utilisation selon la revendication 2, dans laquelle le vecteur est au moins un vecteur viral choisi dans le groupe consistant en les adénovirus, les virus adéno-associés, le virus de l'herpès simplex, les lentivirus, les rétrovirus et les poxvirus ; ou dans laquelle le vecteur est au moins un vecteur non viral choisi dans le groupe consistant en un plasmide, un liposome, un polymère cationique, une micelle, une émulsion et des nanoparticules lipidiques solides.

4. Composition pharmaceutique pour utilisation selon la revendication 1, dans laquelle la GAD est GAD65 ou GAD67.

5. Composition pharmaceutique pour utilisation selon la revendication 1, dans laquelle le gène codant pour GAD est la séquence nucléotidique codant pour la séquence d'acides aminés représentée par SEQ ID NO : 1, SEQ ID NO : 4, SEQ ID NO : 32, SEQ ID NO : 34 ou SEQ ID NO : 36.

6. Composition pharmaceutique pour utilisation selon la revendication 1, dans laquelle le gène codant pour GAD est la séquence nucléotidique représentée par SEQ ID NO : 2, SEQ ID NO : 3, SEQ ID NO : 5, SEQ ID NO : 33, SEQ ID NO : 35 ou SEQ ID NO : 37.

7. Composition pharmaceutique pour utilisation selon la revendication 1, dans laquelle le gène codant pour IL-10 est la séquence nucléotidique codant pour la séquence d'acides aminés représentée par SEQ ID NO : 6, SEQ ID NO : 9, SEQ ID NO : 38, SEQ ID NO : 40 ou SEQ ID NO : 42.

8. Composition pharmaceutique pour utilisation selon la revendication 1, dans laquelle le gène codant pour IL-10 est la séquence nucléotidique représentée par SEQ ID NO : 7, SEQ ID NO : 8, SEQ ID NO : 10, SEQ ID NO : 14, SEQ ID NO : 39, SEQ ID NO : 41 ou SEQ ID NO : 43.

9. Composition pharmaceutique pour utilisation selon la revendication 1, dans laquelle le gène codant pour GDNF est la séquence nucléotidique codant pour la séquence d'acides aminés représentée par SEQ ID NO : 11, SEQ ID NO : 44, SEQ ID NO : 46 ou SEQ ID NO : 48.

10. Composition pharmaceutique pour utilisation selon la revendication 1, dans laquelle le gène codant pour GDNF est la séquence nucléotidique représentée par SEQ ID NO : 12, SEQ ID NO : 13, SEQ ID NO : 45, SEQ ID NO : 47 ou SEQ ID NO : 49.

11. Composition pharmaceutique pour utilisation selon la revendication 1, dans laquelle la douleur est une douleur nociceptive, une douleur psychogène, une douleur inflammatoire, une douleur pathologique, une douleur neuropathique, une douleur cancéreuse, une douleur postopératoire, une douleur de névralgie du trijumeau, une douleur idiopathique, une douleur neuropathique diabétique ou une migraine.

12. Composition pharmaceutique pour utilisation selon la revendication 1, dans laquelle la composition pharmaceutique comprend en outre un support physiologiquement acceptable ; ou dans laquelle la composition pharmaceutique est une injection.

13. Composition pharmaceutique pour utilisation selon la revendication 1, dans laquelle la composition pharmaceutique est formulée pour une administration par injection épidurale ou injection intrathécale.


<110> KOLON LIFE SCIENCE, INC.
<120> COMPOSITION FOR ALLEVIATING OR TREATING PAIN
<130> PCB706093KLS/PCT
<150> KR 2016/0143519
   <151> 2016-10-31
<160> 49
<170> KopatentIn 2.0
<210> 1
   <211> 585
   <212> PRT
   <213> Homo sapiens
<400> 1
   Met Ala Ser Pro Gly Ser Gly Phe Trp Ser Phe Gly Ser Glu Asp Gly
   1               5                   10                  15
   Ser Gly Asp Ser Glu Asn Pro Gly Thr Ala Arg Ala Trp Cys Gln Val
               20                  25                  30
   Ala Gln Lys Phe Thr Gly Gly Ile Gly Asn Lys Leu Cys Ala Leu Leu
           35                  40                  45
   Tyr Gly Asp Ala Glu Lys Pro Ala Glu Ser Gly Gly Ser Gln Pro Pro
       50                  55                  60
   Arg Ala Ala Ala Arg Lys Ala Ala Cys Ala Cys Asp Gln Lys Pro Cys
   65                  70                  75                  80
   Ser Cys Ser Lys Val Asp Val Asn Tyr Ala Phe Leu His Ala Thr Asp
                   85                  90                  95
   Leu Leu Pro Ala Cys Asp Gly Glu Arg Pro Thr Leu Ala Phe Leu Gln
               100                 105                 110
   Asp Val Met Asn Ile Leu Leu Gln Tyr Val Val Lys Ser Phe Asp Arg
           115                 120                 125
   Ser Thr Lys Val Ile Asp Phe His Tyr Pro Asn Glu Leu Leu Gln Glu
       130                 135                 140
   Tyr Asn Trp Glu Leu Ala Asp Gln Pro Gln Asn Leu Glu Glu Ile Leu
   145                 150                 155                 160
   Met His Cys Gln Thr Thr Leu Lys Tyr Ala Ile Lys Thr Gly His Pro
                   165                 170                 175
   Arg Tyr Phe Asn Gln Leu Ser Thr Gly Leu Asp Met Val Gly Leu Ala
               180                 185                 190
   Ala Asp Trp Leu Thr Ser Thr Ala Asn Thr Asn Met Phe Thr Tyr Glu
           195                 200                 205
   Ile Ala Pro Val Phe Val Leu Leu Glu Tyr Val Thr Leu Lys Lys Met
       210                 215                 220
   Arg Glu Ile Ile Gly Trp Pro Gly Gly Ser Gly Asp Gly Ile Phe Ser
   225                 230                 235                 240
   Pro Gly Gly Ala Ile Ser Asn Met Tyr Ala Met Met Ile Ala Arg Phe
                   245                 250                 255
   Lys Met Phe Pro Glu Val Lys Glu Lys Gly Met Ala Ala Leu Pro Arg
               260                 265                 270
   Leu Ile Ala Phe Thr Ser Glu His Ser His Phe Ser Leu Lys Lys Gly
           275                 280                 285
   Ala Ala Ala Leu Gly Ile Gly Thr Asp Ser Val Ile Leu Ile Lys Cys
       290                 295                 300
   Asp Glu Arg Gly Lys Met Ile Pro Ser Asp Leu Glu Arg Arg Ile Leu
   305                 310                 315                 320
   Glu Ala Lys Gln Lys Gly Phe Val Pro Phe Leu Val Ser Ala Thr Ala
                   325                 330                 335
   Gly Thr Thr Val Tyr Gly Ala Phe Asp Pro Leu Leu Ala Val Ala Asp
               340                 345                 350
   Ile Cys Lys Lys Tyr Lys Ile Trp Met His Val Asp Ala Ala Trp Gly
           355                 360                 365
   Gly Gly Leu Leu Met Ser Arg Lys His Lys Trp Lys Leu Ser Gly Val
       370                 375                 380
   Glu Arg Ala Asn Ser Val Thr Trp Asn Pro His Lys Met Met Gly Val
   385                 390                 395                 400
   Pro Leu Gln Cys Ser Ala Leu Leu Val Arg Glu Glu Gly Leu Met Gln
                   405                 410                 415
   Asn Cys Asn Gln Met His Ala Ser Tyr Leu Phe Gln Gln Asp Lys His
               420                 425                 430
   Tyr Asp Leu Ser Tyr Asp Thr Gly Asp Lys Ala Leu Gln Cys Gly Arg
           435                 440                 445
   His Val Asp Val Phe Lys Leu Trp Leu Met Trp Arg Ala Lys Gly Thr
       450                 455                 460
   Thr Gly Phe Glu Ala His Val Asp Lys Cys Leu Glu Leu Ala Glu Tyr
   465                 470                 475                 480
   Leu Tyr Asn Ile Ile Lys Asn Arg Glu Gly Tyr Glu Met Val Phe Asp
                   485                 490                 495
   Gly Lys Pro Gln His Thr Asn Val Cys Phe Trp Tyr Ile Pro Pro Ser
               500                 505                 510
   Leu Arg Thr Leu Glu Asp Asn Glu Glu Arg Met Ser Arg Leu Ser Lys
           515                 520                 525
   Val Ala Pro Val Ile Lys Ala Arg Met Met Glu Tyr Gly Thr Thr Met
       530                 535                 540
   Val Ser Tyr Gln Pro Leu Gly Asp Lys Val Asn Phe Phe Arg Met Val
   545                 550                 555                 560
   Ile Ser Asn Pro Ala Ala Thr His Gln Asp Ile Asp Phe Leu Ile Glu
                   565                 570                 575
   Glu Ile Glu Arg Leu Gly Gln Asp Leu
               580                 585
<210> 2
   <211> 2824
   <212> DNA
   <213> Homo sapiens
<400> 2
   gcggccgccc gcacttcccg cctctggctc gcccgaggac gcgctggcac gcctcccacc      60
   ccctcactct gactccagct ggcgtgcatg gtctgcctcg catcctcacg actcagctcc     120
   ctccctctct cgtgtttttt tcctccgccg ccccctcatt catccccact gggctccctt     180
   tccctcaaat gctctggggc tctccgcgct ttcctgagtc cgggctccga ggacccttag     240
   gtagtcccgg tctcttttaa agctccccgg cttccaaagg gttgccacgt ccctaaaccc     300
   tgtctccagc tcgcatacac acacgcacag acacgcacgt tttctgttcc tgcgtgacac     360
   ccgccctcgc cgctcggccc cgccggtccc cgcgcggtgc cctcctcccg ccacacgggc     420
   acgcacgcgc gcgcagggcc aagcccgagg cagctcgccc gcagctcgca ctcgcaggcg     480
   acctgctcca gtctccaaag ccgatggcat ctccgggctc tggcttttgg tctttcgggt     540
   cggaagatgg ctctggggat tccgagaatc ccggcacagc gcgagcctgg tgccaagtgg     600
   ctcagaagtt cacgggcggc atcggaaaca aactgtgcgc cctgctctac ggagacgccg     660
   agaagccggc ggagagcggc gggagccaac ccccgcgggc cgccgcccgg aaggccgcct     720
   gcgcctgcga ccagaagccc tgcagctgct ccaaagtgga tgtcaactac gcgtttctcc     780
   atgcaacaga cctgctgccg gcgtgtgatg gagaaaggcc cactttggcg tttctgcaag     840
   atgttatgaa cattttactt cagtatgtgg tgaaaagttt cgatagatca accaaagtga     900
   ttgatttcca ttatcctaat gagcttctcc aagaatataa ttgggaattg gcagaccaac     960
   cacaaaattt ggaggaaatt ttgatgcatt gccaaacaac tctaaaatat gcaattaaaa    1020
   cagggcatcc tagatacttc aatcaacttt ctactggttt ggatatggtt ggattagcag    1080
   cagactggct gacatcaaca gcaaatacta acatgttcac ctatgaaatt gctccagtat    1140
   ttgtgctttt ggaatatgtc acactaaaga aaatgagaga aatcattggc tggccagggg    1200
   gctctggcga tgggatattt tctcccggtg gcgccatatc taacatgtat gccatgatga    1260
   tcgcacgctt taagatgttc ccagaagtca aggagaaagg aatggctgct cttcccaggc    1320
   tcattgcctt cacgtctgaa catagtcatt tttctctcaa gaagggagct gcagccttag    1380
   ggattggaac agacagcgtg attctgatta aatgtgatga gagagggaaa atgattccat    1440
   ctgatcttga aagaaggatt cttgaagcca aacagaaagg gtttgttcct ttcctcgtga    1500
   gtgccacagc tggaaccacc gtgtacggag catttgaccc cctcttagct gtcgctgaca    1560
   tttgcaaaaa gtataagatc tggatgcatg tggatgcagc ttggggtggg ggattactga 1620
   tgtcccgaaa acacaagtgg aaactgagtg gcgtggagag ggccaactct gtgacgtgga 1680
   atccacacaa gatgatggga gtccctttgc agtgctctgc tctcctggtt agagaagagg 1740
   gattgatgca gaattgcaac caaatgcatg cctcctacct ctttcagcaa gataaacatt 1800
   atgacctgtc ctatgacact ggagacaagg ccttacagtg cggacgccac gttgatgttt 1860
   ttaaactatg gctgatgtgg agggcaaagg ggactaccgg gtttgaagcg catgttgata 1920
   aatgtttgga gttggcagag tatttataca acatcataaa aaaccgagaa ggatatgaga 1980
   tggtgtttga tgggaagcct cagcacacaa atgtctgctt ctggtacatt cctccaagct 2040
   tgcgtactct ggaagacaat gaagagagaa tgagtcgcct ctcgaaggtg gctccagtga 2100
   ttaaagccag aatgatggag tatggaacca caatggtcag ctaccaaccc ttgggagaca 2160
   aggtcaattt cttccgcatg gtcatctcaa acccagcggc aactcaccaa gacattgact 2220
   tcctgattga agaaatagaa cgccttggac aagatttata ataaccttgc tcaccaagct 2280
   gttccacttc tctagagaac atgccctcag ctaagccccc tactgagaaa cttcctttga 2340
   gaattgtgcg acttcacaaa atgcaaggtg aacaccactt tgtctctgag aacagacgtt 2400
   accaattatg gagtgtcacc agctgccaaa atcgtaggtg ttggctctgc tggtcactgg 2460
   agtagttgct actcttcaga atatggacaa agaaggcaca ggtgtaaata tagtagcagg 2520
   atgaggaacc tcaaactggg tatcattttg cacgtgctct tctgttctca aatgctaaat 2580
   gcaaacactg tgtatttatt agttaggtgt gccaaactac cgttcccaaa ttggtgtttc 2640
   tgaatgacat caacattccc ccaacattac tccattacta aagacagaaa aaaataaaaa 2700
   cataaaatat acaaacatgt ggcaacctgt tcttcctacc aaatataaac ttgtgtatga 2760
   tccaagtatt ttatctgtgt tgtctctcta aacccaaata aatgtgtaaa tgtggacaca 2820
   tctc 2824
<210> 3
   <211> 1758
   <212> DNA
   <213> Artificial Sequence
<220>
   <223> Optimized human GAD65
<400> 3
   atggcatctc cgggctccgg cttttggtcc ttcgggtcgg aagatggctc aggggattcc 60
   gagaatcccg gcacagcgcg ggcctggtgt caagtggctc agaagttcac gggcggcatc 120
   ggaaacaaac tgtgtgccct gctctacggc gacgccgaga agcccgcaga gagcggcggg 180
   agccaacccc cgcgggccgc cgcccggaag gccgcctgcg cctgtgacca gaagccctgc 240
   tcatgcagca aggtagatgt caactacgcg tttctccatg ccacagatct gctgccggct 300
   tgcgacggtg aaaggcccac tttggccttt ctgcaggatg ttatgaacat tctgctgcag 360
   tacgtggtga aaagtttcga ccggtcaacc aaagtgatcg actttcacta tcctaatgaa 420
   cttctccagg agtacaattg ggagctggct gaccagccac agaacctgga ggaaatcttg 480
   atgcattgcc aaactactct aaaatatgca attaaaacag gccatcctag atacttcaac 540
   cagctttcta ccggtttgga tatggtgggg ctggcagccg actggctgac atccaccgca 600
   aataccaaca tgttcaccta tgagatcgct cctgtcttcg tgcttttgga atacgtcacc 660
   ctaaagaaga tgcgtgaaat cattggctgg ccaggaggct ctggtgatgg tatattttct 720
   cccggcggcg cgatctctaa catgtatgcc atgatgatcg cacgctttaa gatgttccca 780
   gaagtcaagg agaaaggaat ggctgctctt cccaggctca ttgccttcac gagtgaacac 840
   agtcactttt ccctcaagaa gggggctgcc gccttaggga tcggaacaga cagcgtgatt 900
   ctgataaagt gcgacgagag agggaaaatg attccatctg atcttgagag aaggattctt 960
   gaagccaaac agaaagggtt tgtccctttc ctcgtgagtg ccacagctgg aaccaccgtg 1020
   tacggcgcat ttgaccccct cttagctgtc gcggatatat gtaagaagta taagatctgg 1080
   atgcacgtgg atgctgcttg gggtggggga ttactgatgt ccaggaaaca caagtggaaa 1140
   ctgtctggcg tggagcgcgc caacagcgtg acgtggaatc cacacaaaat gatgggagtc 1200
   cctttgcagt gctctgctct cctggttcga gaagagggac tgatgcagaa ttgcaaccaa 1260
   atgcatgcct cctacctctt tcagcaggat aaacattatg acctgtctta cgacactggt 1320
   gacaaggccc tgcagtgtgg gcgccacgtt gatgtattca agctatggct gatgtggagg 1380
   gcaaagggga ctaccggttt tgaagcccat gttgacaaat gtctggagtt ggcagagtat 1440
   ttatacaata tcataaaaaa ccgagaagga tatgagatgg tgtttgatgg caagcctcag 1500
   cacacaaatg tctgcttctg gtacatccct cccagcctac gtactctgga ggacaacgaa 1560
   gagagaatga gtcgcctctc gaaggtggct ccagtgatta aagccagaat gatggagtat 1620
   ggaaccacaa tggtcagcta ccaacccttg ggggacaagg taaatttctt ccgcatggtc 1680
   atctcaaacc cagcggcaac tcaccaagac attgatttcc tgattgaaga gatcgagcgg 1740
   ctcggccagg atctgtga 1758
<210> 4
   <211> 594
   <212> PRT
   <213> Homo sapiens
<400> 4
   Met Ala Ser Ser Thr Pro Ser Ser Ser Ala Thr Ser Ser Asn Ala Gly
     1               5                  10                  15
   Ala Asp Pro Asn Thr Thr Asn Leu Arg Pro Thr Thr Tyr Asp Thr Trp
   20 25 30
   Cys Gly Val Ala His Gly Cys Thr Arg Lys Leu Gly Leu Lys Ile Cys
   35 40 45
   Gly Phe Leu Gln Arg Thr Asn Ser Leu Glu Glu Lys Ser Arg Leu Val
   50 55 60
   Ser Ala Phe Lys Glu Arg Gln Ser Ser Lys Asn Leu Leu Ser Cys Glu
   65 70 75 80
   Asn Ser Asp Arg Asp Ala Arg Phe Arg Arg Thr Glu Thr Asp Phe Ser
   85 90 95
   Asn Leu Phe Ala Arg Asp Leu Leu Pro Ala Lys Asn Gly Glu Glu Gln
   100 105 110
   Thr Val Gln Phe Leu Leu Glu Val Val Asp Ile Leu Leu Asn Tyr Val
   115 120 125
   Arg Lys Thr Phe Asp Arg Ser Thr Lys Val Leu Asp Phe His His Pro
   130 135 140
   His Gln Leu Leu Glu Gly Met Glu Gly Phe Asn Leu Glu Leu Ser Asp
   145 150 155 160
   His Pro Glu Ser Leu Glu Gln Ile Leu Val Asp Cys Arg Asp Thr Leu
   165 170 175
   Lys Tyr Gly Val Arg Thr Gly His Pro Arg Phe Phe Asn Gln Leu Ser
   180 185 190
   Thr Gly Leu Asp Ile Ile Gly Leu Ala Gly Glu Trp Leu Thr Ser Thr
   195 200 205
   Ala Asn Thr Asn Met Phe Thr Tyr Glu Ile Ala Pro Val Phe Val Leu
   210 215 220
   Met Glu Gln Ile Thr Leu Lys Lys Met Arg Glu Ile Val Gly Trp Ser
   225 230 235 240
   Ser Lys Asp Gly Asp Gly Ile Phe Ser Pro Gly Gly Ala Ile Ser Asn
   245 250 255
   Met Tyr Ser Ile Met Ala Ala Arg Tyr Lys Tyr Phe Pro Glu Val Lys
   260 265 270
   Thr Lys Gly Met Ala Ala Val Pro Lys Leu Val Leu Phe Thr Ser Glu
   275 280 285
   Gln Ser His Tyr Ser Ile Lys Lys Ala Gly Ala Ala Leu Gly Phe Gly
   290 295 300
   Thr Asp Asn Val Ile Leu Ile Lys Cys Asn Glu Arg Gly Lys Ile Ile
   305 310 315 320
   Pro Ala Asp Phe Glu Ala Lys Ile Leu Glu Ala Lys Gln Lys Gly Tyr
   325 330 335
   Val Pro Phe Tyr Val Asn Ala Thr Ala Gly Thr Thr Val Tyr Gly Ala
   340 345 350
   Phe Asp Pro Ile Gln Glu Ile Ala Asp Ile Cys Glu Lys Tyr Asn Leu
               355                 360                 365
   Trp Leu His Val Asp Ala Ala Trp Gly Gly Gly Leu Leu Met Ser Arg
       370                 375                 380
   Lys His Arg His Lys Leu Asn Gly Ile Glu Arg Ala Asn Ser Val Thr
   385                 390                 395                 400
   Trp Asn Pro His Lys Met Met Gly Val Leu Leu Gln Cys Ser Ala Ile
                   405                 410                 415
   Leu Val Lys Glu Lys Gly Ile Leu Gln Gly Cys Asn Gln Met Cys Ala
               420                 425                 430
   Gly Tyr Leu Phe Gln Pro Asp Lys Gln Tyr Asp Val Ser Tyr Asp Thr
           435                 440                 445
   Gly Asp Lys Ala Ile Gln Cys Gly Arg His Val Asp Ile Phe Lys Phe
       450                 455                 460
   Trp Leu Met Trp Lys Ala Lys Gly Thr Val Gly Phe Glu Asn Gln Ile
   465                 470                 475                 480
   Asn Lys Cys Leu Glu Leu Ala Glu Tyr Leu Tyr Ala Lys Ile Lys Asn
                   485                 490                 495
   Arg Glu Glu Phe Glu Met Val Phe Asn Gly Glu Pro Glu His Thr Asn
               500                 505                 510
   Val Cys Phe Trp Tyr Ile Pro Gln Ser Leu Arg Gly Val Pro Asp Ser
           515                 520                 525
   Pro Gln Arg Arg Glu Lys Leu His Lys Val Ala Pro Lys Ile Lys Ala
       530                 535                 540
   Leu Met Met Glu Ser Gly Thr Thr Met Val Gly Tyr Gln Pro Gln Gly
   545                 550                 555                 560
   Asp Lys Ala Asn Phe Phe Arg Met Val Ile Ser Asn Pro Ala Ala Thr
                   565                 570                 575
   Gln Ser Asp Ile Asp Phe Leu Ile Glu Glu Ile Glu Arg Leu Gly Gln
               580                 585                 590
   Asp Leu
<210> 5
   <211> 1784
   <212> DNA
   <213> Homo sapiens
<400> 5
   atggcgtctc gaccccatct tcgtccgcaa cctcctcgaa cgcgggagcg gaccccaata      60
   ccactaacct gcgccccaca acgtacgata cctggtgcgg cgtggcccat ggatgcacca     120
   gaaaactggg gctcaagatc tgcggcttct tgcaaaggac caacagcctg gaagagaaga     180
   gtcgccttgt gagtgccttc aaggagaggc aatcctccaa gaacctgctt tcctgtgaaa     240
   acagcgaccg ggatgcccgc ttccggcgca cagagactga cttctctaat ctgtttgcta     300
   gagatctgct tccggctaag aacggtgagg agcaaaccgt gcaattcctc ctggaagtgg     360
   tggacatact cctcaactat gtccgcaaga catttgatcg ctccaccaag gtgctggact     420
   ttcatcaccc acaccagttg ctggaaggca tggagggctt caacttggag ctctctgacc     480
   accccgagtc cctggagcag atcctggttg actgcagaga caccttgaag tatggggttc     540
   gcacaggtca tcctcgattt ttcaaccagc tctccactgg attggatatt attggcctag     600
   ctggagaatg gctgacatca acggccaata ccaacatgtt tacatatgaa attgcaccag     660
   tgtttgtcct catggaacaa ataacactta agaagatgag agagatagtt ggatggtcaa     720
   gtaaagatgg tgatgggata ttttctcctg ggggcgccat atccaacatg tacagcatca      780
   tggctgctcg ctacaagtac ttcccggaag ttaagacaaa gggcatggcg gctgtgccta     840
   aactggtcct cttcacctca gaacagagtc actattccat aaagaaagct ggggctgcac     900
   ttggctttgg aactgacaat gtgattttga taaagtgcaa tgaaaggggg aaaataattc     960
   cagctgattt tgaggcaaaa attcttgaag ccaaacagaa gggatatgtt cccttttatg   1020
   tcaatgcaac tgctggcacg actgtttatg gagcttttga tccgatacaa gagattgcag    1080
   atatatgtga gaaatataac ctttggttgc atgtcgatgc tgcctgggga ggtgggctgc    1140
   tcatgtccag gaagcaccgc cataaactca acggcataga aagggccaac tcagtcacct    1200
   ggaaccctca caagatgatg ggcgtgctgt tgcagtgctc tgccattctc gtcaaggaaa    1260
   agggtatact ccaaggatgc aaccagatgt gtgcaggata cctcttccag ccagacaagc    1320
   agtatgatgt ctcctacgac accggggaca aggcaattca gtgtggccgc cacgtggata    1380
   tcttcaagtt ctggctgatg tggaaagcaa agggcacagt gggatttgaa aaccagatca    1440
   acaaatgcct ggaactggct gaatacctct atgccaagat taaaaacaga gaagaatttg    1500
   agatggtttt caatggcgag cctgagcaca caaacgtctg tttttggtat attccacaaa    1560
   gcctcagggg tgtgccagac agccctcaac gacgggaaaa gctacacaag gtggctccaa    1620
   aaatcaaagc cctgatgatg gagtcaggta cgaccatggt tggctaccag ccccaagggg    1680
   acaaggccaa cttcttccgg atggtcatct ccaacccagc cgctacccag tctgacattg    1740
   acttcctcat tgaggagata gaaagactgg gccaggatct gtaa                     1784
<210> 6
   <211> 178
   <212> PRT
   <213> rattus norvegicus
<400> 6
   Met Pro Gly Ser Ala Leu Leu Cys Cys Leu Leu Leu Leu Ala Gly Val
    1               5                  10                  15
   Lys Thr Ser Lys Gly His Ser Ile Arg Gly Asp Asn Asn Cys Thr His
               20                  25                  30
   Phe Pro Val Ser Gln Thr His Met Leu Arg Glu Leu Arg Ala Ala Phe
           35                  40                  45
   Ser Gln Val Lys Thr Phe Phe Gln Lys Lys Asp Gln Leu Asp Asn Ile
       50                  55                  60
   Leu Leu Thr Asp Ser Leu Leu Gln Asp Phe Lys Gly Tyr Leu Gly Cys
   65                  70                  75                  80
   Gln Ala Leu Ser Glu Met Ile Lys Phe Tyr Leu Val Glu Val Met Pro
                   85                  90                  95
   Gln Ala Glu Asn His Gly Pro Glu Ile Lys Glu His Leu Asn Ser Leu
               100                 105                 110
   Gly Glu Lys Leu Lys Thr Leu Trp Ile Gln Leu Arg Arg Cys His Arg
           115                 120                 125
   Phe Leu Pro Cys Glu Asn Lys Ser Lys Ala Val Glu Gln Val Lys Asn
       130                 135                 140
   Asp Phe Asn Lys Leu Gln Asp Lys Gly Val Tyr Lys Ala Met Asn Glu
   145                 150                 155                 160
   Phe Asp Ile Phe Ile Asn Cys Ile Glu Ala Tyr Val Thr Leu Lys Met
                   165                 170                 175
   Lys Asn
<210> 7
   <211> 682
   <212> DNA
   <213> rattus norvegicus
<400> 7
   catgcctggc tcagcactgc tatgttgcct gctcttactg gctggagtga agaccagcaa      60
   aggccattcc atccggggtg acaataactg cacccacttc ccagtcagcc agacccacat     120
   gctccgagag ctgagggctg ccttcagtca agtgaagact ttctttcaaa agaaggacca     180
   gctggacaac atactgctga cagattcctt actgcaggac tttaagggtt acttgggttg     240
   ccaagccttg tcagaaatga tcaagtttta cctggtagaa gtgatgcccc aggcagagaa     300
   ccatggccca gaaatcaagg agcatttgaa ttccctggga gagaagctga agaccctctg     360
   gatacagctg cgacgctgtc atcgatttct cccctgtgag aataaaagca aggcagtgga     420
   gcaggtgaag aatgatttta ataagctcca agacaaaggt gtctacaagg ccatgaatga     480
   gtttgacatc ttcatcaact gcatagaagc ctacgtgaca ctcaaaatga aaaattgaac     540
   cacccggcat ctactggact gcaggacata aatagagctt ctaaatctga tccagagatc     600
   ttagctaacg ggagcaactc cttggaaaac ctcgtttgta cctctctcca aaatatttat     660
   tacctctgat acctcagttc cc                                              682
<210> 8
   <211> 537
   <212> DNA
   <213> Artificial Sequence
<220>
   <223> Optimized rat IL-10
<400> 8
   atgcctggct cagccctgct atgttgcctt ctcctgctgg cgggagtcaa gacaagcaag      60
   ggccattcca tccggggaga taataactgc acccacttcc cagtctctca aacccacatg     120
   ttgcgagagc tgagggctgc cttcagtcag gtgaagacgt tcttccagaa gaaggaccag     180
   ctggacaaca ttctgctgac tgacagcctg ctgcaggatt tcaagggtta tttggggtgt     240
   caagccctgt ctgaaatgat caagttttac ctggtagaag tgatgcccca ggcagagaat     300
   catggccccg agatcaagga gcacctcaac tccctggggg agaagctgaa gaccctgtgg     360
   attcagctga ggcgctgcca cagatttctc ccctgtgaaa acaagagcaa ggcagtggag     420
   caggtgaaga acgattttaa taagctccag gacaagggcg tctacaaggc catgaacgag     480
   ttcgacatct ttatcaactg catagaagct tacgttacac tcaagatgaa gaattga        537
<210> 9
   <211> 178
   <212> PRT
   <213> Homo sapiens
<400> 9
   Met His Ser Ser Ala Leu Leu Cys Cys Leu Val Leu Leu Thr Gly Val
    1               5                  10                  15
   Arg Ala Ser Pro Gly Gln Gly Thr Gln Ser Glu Asn Ser Cys Thr His
               20                  25                  30
   Phe Pro Gly Asn Leu Pro Asn Met Leu Arg Asp Leu Arg Asp Ala Phe
           35                  40                  45
   Ser Arg Val Lys Thr Phe Phe Gln Met Lys Asp Gln Leu Asp Asn Leu
       50                  55                  60
   Leu Leu Lys Glu Ser Leu Leu Glu Asp Phe Lys Gly Tyr Leu Gly Cys
    65                 70                  75                      80
   Gln Ala Leu Ser Glu Met Ile Gln Phe Tyr Leu Glu Glu Val Met Pro
                   85                  90                  95
   Gln Ala Glu Asn Gln Asp Pro Asp Ile Lys Ala His Val Asn Ser Leu
               100                 105                 110
   Gly Glu Asn Leu Lys Thr Leu Arg Leu Arg Leu Arg Arg Cys His Arg
           115                 120                 125
   Phe Leu Pro Cys Glu Asn Lys Ser Lys Ala Val Glu Gln Val Lys Asn
       130                 135                 140
   Ala Phe Asn Lys Leu Gln Glu Lys Gly Ile Tyr Lys Ala Met Ser Glu
   145                 150                 155                 160
   Phe Asp Ile Phe Ile Asn Tyr Ile Glu Ala Tyr Met Thr Met Lys Ile
                   165                 170                 175
   Arg Asn
<210> 10
   <211> 1600
   <212> DNA
   <213> Homo sapiens
<400> 10
   aaaccacaag acagacttgc aaaagaaggc atgcacagct cagcactgct ctgttgcctg      60
   gtcctcctga ctggggtgag ggccagccca ggccagggca cccagtctga gaacagctgc     120
   acccacttcc caggcaacct gcctaacatg cttcgagatc tccgagatgc cttcagcaga     180
   gtgaagactt tctttcaaat gaaggatcag ctggacaact tgttgttaaa ggagtccttg     240
   ctggaggact ttaagggtta cctgggttgc caagccttgt ctgagatgat ccagttttac     300
   ctggaggagg tgatgcccca agctgagaac caagacccag acatcaaggc gcatgtgaac     360
   tccctggggg agaacctgaa gaccctcagg ctgaggctac ggcgctgtca tcgatttctt     420
   ccctgtgaaa acaagagcaa ggccgtggag caggtgaaga atgcctttaa taagctccaa     480
   gagaaaggca tctacaaagc catgagtgag tttgacatct tcatcaacta catagaagcc     540
   tacatgacaa tgaagatacg aaactgagac atcagggtgg cgactctata gactctagga     600
   cataaattag aggtctccaa aatcggatct ggggctctgg gatagctgac ccagcccctt     660
   gagaaacctt attgtacctc tcttatagaa tatttattac ctctgatacc tcaaccccca    720
   tttctattta tttactgagc ttctctgtga acgatttaga aagaagccca atattataat     780
   ttttttcaat atttattatt ttcacctgtt tttaagctgt ttccataggg tgacacacta     840
   tggtatttga gtgttttaag ataaattata agttacataa gggaggaaaa aaaatgttct     900
   ttggggagcc aacagaagct tccattccaa gcctgaccac gctttctagc tgttgagctg     960
   ttttccctga cctccctcta atttatcttg tctctgggct tggggcttcc taactgctac    1020
   aaatactctt aggaagagaa accagggagc ccctttgatg attaattcac cttccagtgt    1080
   ctcggaggga ttcccctaac ctcattcccc aaccacttca ttcttgaaag ctgtggccag    1140
   cttgttattt ataacaacct aaatttggtt ctaggccggg cgcggtggct cacgcctgta    1200
   atcccagcac tttgggaggc tgaggcgggt ggatcacttg aggtcaggag ttcctaacca    1260
   gcctggtcaa catggtgaaa ccccgtctct actaaaaata caaaaattag ccgggcatgg    1320
   tggcgcgcac ctgtaatccc agctacttgg gaggctgagg caagagaatt gcttgaaccc    1380
   aggagatgga agttgcagtg agctgatatc atgcccctgt actccagcct gggtgacaga    1440
   gcaagactct gtctcaaaaa ataaaaataa aaataaattt ggttctaata gaactcagtt    1500
   ttaactagaa tttattcaat tcctctggga atgttacatt gtttgtctgt cttcatagca    1560
   gattttaatt ttgaataaat aaatgtatct tattcacatc                           1600
<210> 11
   <211> 185
   <212> PRT
   <213> Homo sapiens
<400> 11
   Met Lys Leu Trp Asp Val Val Ala Val Cys Leu Val Leu Leu His Thr
   1               5                   10                  15
   Ala Ser Ala Phe Pro Leu Pro Ala Ala Asn Met Pro Glu Asp Tyr Pro
               20                  25                  30
   Asp Gln Phe Asp Asp Val Met Asp Phe Ile Gln Ala Thr Ile Lys Arg
           35                  40                  45
   Leu Lys Arg Ser Pro Asp Lys Gln Met Ala Val Leu Pro Arg Arg Glu
       50                  55                  60
   Arg Asn Arg Gln Ala Ala Ala Ala Asn Pro Glu Asn Ser Arg Gly Lys
   65                  70                  75                  80
   Gly Arg Arg Gly Gln Arg Gly Lys Asn Arg Gly Cys Val Leu Thr Ala
                   85                  90                  95
   Ile His Leu Asn Val Thr Asp Leu Gly Leu Gly Tyr Glu Thr Lys Glu
               100                 105                 110
   Glu Leu Ile Phe Arg Tyr Cys Ser Gly Ser Cys Asp Ala Ala Glu Thr
           115                 120                 125
   Thr Tyr Asp Lys Ile Leu Lys Asn Leu Ser Arg Asn Arg Arg Leu Val
       130                 135                 140
   Ser Asp Lys Val Gly Gln Ala Cys Cys Arg Pro Ile Ala Phe Asp Asp
   145                 150                 155                 160
   Asp Leu Ser Phe Leu Asp Asp Asn Leu Val Tyr His Ile Leu Arg Lys
                   165                 170                 175
   His Ser Ala Lys Arg Cys Gly Cys Ile
               180                 185
<210> 12
   <211> 558
   <212> DNA
   <213> Homo sapiens
<400> 12
   atgaagttat gggatgtcgt ggctgtctgc ctggtgctgc tccacaccgc gtccgccttc        60
   ccgctgcccg ccgcaaatat gccagaggat tatcctgatc agttcgatga tgtcatggat       120
   tttattcaag ccaccattaa aagactgaaa aggtcaccag ataaacaaat ggcagtgctt       180
   cctagaagag agcggaatcg gcaggctgca gctgccaacc cagagaattc cagaggaaaa       240
   ggtcggagag gccagagggg caaaaaccgg ggttgtgtct taactgcaat acatttaaat 300
   gtcactgact tgggtctggg ctatgaaacc aaggaggaac tgatttttag gtactgcagc 360
   ggctcttgcg atgcagctga gacaacgtac gacaaaatat tgaaaaactt atccagaaat 420
   agaaggctgg tgagtgacaa agtagggcag gcatgttgca gacccatcgc ctttgatgat 480
   gacctgtcgt ttttagatga taacctggtt taccatattc taagaaagca ttccgctaaa 540
   aggtgtggat gtatctga 558
<210> 13
   <211> 558
   <212> DNA
   <213> Artificial Sequence
<220>
   <223> optimized human GDNF
<400> 13
   atgaaacttt gggacgtggt ggctgtctgc ctggtgctcc tccacaccgc cagtgcgttt 60
   ccgctgcccg ccgctaacat gccagaggat tatcctgatc agttcgatga tgttatggac 120
   ttcattcaag ccacaatcaa gcggctgaaa cgatcaccag ataaacagat ggcagtgctt 180
   cctcgccgcg agcgtaatcg gcaggctgca gcagccaatc ccgagaattc ccgaggaaaa 240
   gggcgcaggg gtcagagggg caagaaccgg gggtgtgtcc tgactgcaat acatttaaac 300
   gtgactgact tgggtctggg ctatgagacc aaggaagaac tcattttcag gtactgcagc 360
   ggctcttgcg atgccgcgga aacaacgtac gacaaaatct tgaagaacct ctccagaaac 420
   agaaggctgg tgagtgacaa ggtaggacag gcctgttgca gacccatcgc ctttgacgac 480
   gatctgagct ttctggatga caatctggtt taccacatcc tacggaagca ttctgctaaa 540
   agatgtggat gtatttga 558
<210> 14
   <211> 537
   <212> DNA
   <213> Artificial Sequence
<220>
   <223> optimized human IL-10
<400> 14
   atgcacagct cagcactgct gtgctgcctg gtcctgctga caggggtgag ggcaagccca 60
   ggccagggaa cccaatctga gaacagctgc acccacttcc ctggcaatct gcctaacatg 120
   ctgcgcgacc tccgagatgc cttcagcaga gtgaagactt ttttccagat gaaggatcag 180
   ctggacaacc tgctgctgaa ggagtccctc ctggaggact ttaagggcta cctgggatgc 240
   caggccctgt ctgagatgat ccaattctac ctggaagaag ttatgcccca ggctgagaac 300
   caggacccag acattaaggc ccatgtcaac tccctggggg aaaatctgaa gaccctcagg 360
   ctgcggctac ggcgctgtca ccgttttctg ccctgtgaga ataagagcaa ggctgtggag 420
   caggtgaaga acgccttcaa taagctccag gagaagggta tctacaaagc gatgagtgaa 480
   tttgatatct tcattaatta tatagaagct tatatgacaa tgaaaatcag aaactga 537
<210> 15
   <211> 28
   <212> DNA
   <213> Artificial Sequence
<220>
   <223> Forward primer for amplifying CMV promoter (F-JDK)
<400> 15
   ttcggccgtc gaggagcttg gcccattg 28
<210> 16
   <211> 36
   <212> DNA
   <213> Artificial Sequence
<220>
   <223> Reverse primer for amplifying CMV promoter (R-JDK)
<400> 16
   gacgtcgacc tagctagcga attcggggcc gcggag 36
<210> 17
   <211> 34
   <212> DNA
   <213> Artificial Sequence
<220>
   <223> Forward primer for amplifying SV40pA promoter (F-SV40pA)
<400> 17
   ccatcgatca gacatgataa gatacattga tgag 34
<210> 18
   <211> 43
   <212> DNA
   <213> Artificial Sequence
<220>
   <223> Reverse primer for amplifying SV40pA promoter (R-SV40pA)
<400> 18
   gacgtcgacg cggccgctac cacatttgta gaggttttac ttg 43
<210> 19
   <211> 28
   <212> DNA
   <213> Artificial Sequence
<220>
   <223> Forward primer for amplifying Kanamycin resistant gene (F-Kan)
<400> 19
   aggcgccatg agccatattc aacgggaa 28
<210> 20
   <211> 29
   <212> DNA
   <213> Artificial Sequence
<220>
   <223> Reverse primer for amplifying Kanamycin resistant gene (R-Kan)
<400> 20
   ttcatgatta gaaaaactca tcgagcatc 29
<210> 21
   <211> 28
   <212> DNA
   <213> Artificial Sequence
<220>
   <223> Forward primer for amplifying LITR and CMV (F-ITR)
<400> 21
   atggcgcgcc cctggccttt tgctggcc 28
<210> 22
   <211> 28
   <212> DNA
   <213> Artificial Sequence
<220>
   <223> Reverse primer for amplifying SV40pA and RITR (R-ITR)
<400> 22
   atggatccgc tagtaaatac cgcatcag 28
<210> 23
   <211> 20
   <212> DNA
   <213> Artificial Sequence
<220>
   <223> Forward primer for amplifying rIL-10 (F-rIL-10)
<400> 23
   ccgctagcgc caccatgcct 20
<210> 24
   <211> 39
   <212> DNA
   <213> Artificial Sequence
<220>
   <223> Reverse primer for amplifying rIL-10 (R-rIL-10)
<400> 24
   gacgtcgacg ccatcgatgg cttaattaat caattcttc 39
<210> 25
   <211> 40
   <212> DNA
   <213> Artificial Sequence
<220>
   <223> Forward primer for WPRE_Pac1_F
<400> 25
   ggtggtttaa ttaaaatcaa cctctggatt acaaaatttg 40
<210> 26
   <211> 30
   <212> DNA
   <213> Artificial Sequence
<220>
   <223> Reverse primer for WPRE_modi_Hpa1_R
<400> 26
   ggtggtgtta acgacaacac cacggaattg 30
<210> 27
   <211> 43
   <212> DNA
   <213> Artificial Sequence
<220>
   <223> Forward primer for SV40-CMV-sCAG-bGHpA-Infu-F
<400> 27
   cctgcggccg gtcgactacc acatttgtag aggttttact tgc 43
<210> 28
   <211> 35
   <212> DNA
   <213> Artificial Sequence
<220>
   <223> Reverse primer for SV40-CMV-sCAG-bGHpA-Infu-R
<400> 28
   aataatcaat gtcgactcga ggagcttggc ccatt 35
<210> 29
   <211> 42
   <212> DNA
   <213> Artificial Sequence
<220>
   <223> nucleotide sequence of Stuffer scramble
<400> 29
   gtcgacggta tcgataagct tgatatcgaa ttcctgcagc cc 42
<210> 30
   <211> 46
   <212> DNA
   <213> Artificial Sequence
<220>
   <223> Forward primer for Stuffer_scramble_F
<400> 30
   ctaggtcgac ggtatcgata agcttgatat cgaattcctg cagccc 46
<210> 31
   <211> 46
   <212> DNA
   <213> Artificial Sequence
<220>
   <223> Reverse primer for Stuffer_scramble_R
<400> 31
   ctaggggctg caggaattcg atatcaagct tatcgatacc gtcgac 46
<210> 32
   <211> 585
   <212> PRT
   <213> Canis lupus
<400> 32
   Met Ala Ser Pro Gly Ser Gly Phe Trp Ser Phe Gly Ser Glu Asp Gly
   1               5                   10                  15
   Ser Gly Asp Pro Glu Asn Pro Ser Thr Ala Arg Ala Trp Cys Gln Val
               20                  25                  30
   Ala Gln Lys Phe Thr Gly Gly Ile Gly Asn Lys Leu Cys Ala Leu Leu
           35                  40                  45
   Tyr Gly Asp Ala Glu Lys Pro Ala Glu Ser Gly Gly Ser Glu Pro Pro
       50                  55                  60
   Arg Ala Thr Ser Arg Lys Ala Ala Cys Ala Cys Asn Gln Lys Pro Cys
   65                  70                  75                  80
   Ser Cys Pro Lys Ala Glu Val Asn Tyr Ala Phe Leu His Ala Thr Asp
                   85                  90                  95
   Leu Leu Pro Ala Cys Asp Gly Glu Arg Pro Thr Leu Ala Phe Leu Gln
               100                 105                 110
   Asp Val Met Asp Ile Leu Leu Gln Tyr Val Val Lys Ser Phe Asp Arg
           115                 120                 125
   Ser Thr Lys Val Ile Asp Phe His Tyr Pro Asn Glu Leu Leu Gln Glu
       130                 135                 140
   Tyr Asn Trp Glu Leu Ala Asp Gln Pro Gln Asn Leu Glu Glu Ile Leu
   145                 150                 155                 160
   Met His Cys Gln Thr Thr Leu Lys Tyr Ala Ile Lys Thr Gly His Pro
                   165                 170                 175
   Arg Tyr Phe Asn Gln Leu Ser Thr Gly Leu Asp Met Val Gly Leu Ala
               180                 185                 190
   Ala Asp Trp Leu Thr Ser Thr Ala Asn Thr Asn Met Phe Thr Tyr Glu
           195                 200                 205
   Ile Ala Pro Val Phe Val Leu Leu Glu Tyr Val Thr Leu Lys Lys Met
       210                 215                 220
   Arg Glu Ile Ile Gly Trp Pro Gly Gly Ser Gly Asp Gly Ile Phe Ser
   225                 230                 235                 240
   Pro Gly Gly Ala Ile Ser Asn Met Tyr Ala Met Leu Ile Ala Arg Phe
                   245                 250                 255
   Lys Met Phe Pro Glu Val Lys Glu Lys Gly Met Ala Ala Val Pro Arg
               260                 265                 270
   Leu Ile Ala Phe Thr Ser Glu His Ser His Phe Ser Leu Lys Lys Gly
           275                 280                 285
   Ala Ala Ala Leu Gly Ile Gly Thr Asp Ser Val Ile Leu Ile Lys Cys
       290                 295                 300
   Asp Glu Arg Gly Lys Met Val Pro Ser Asp Leu Glu Arg Arg Ile Leu
   305                 310                 315                 320
   Glu Ala Lys Gln Lys Gly Phe Val Pro Phe Leu Val Ser Ala Thr Ala
                   325                 330                 335
   Gly Thr Thr Val Tyr Gly Ala Phe Asp Pro Leu Leu Ala Val Ala Asp
               340                 345                 350
   Ile Cys Lys Lys Tyr Lys Ile Trp Met His Val Asp Ala Ala Trp Gly
           355                 360                 365
   Gly Gly Leu Leu Met Ser Arg Lys His Lys Trp Lys Leu Ser Gly Val
       370                 375                 380
   Glu Arg Ala Asn Ser Val Thr Trp Asn Pro His Lys Met Met Gly Val
   385                 390                 395                 400
   Pro Leu Gln Cys Ser Ala Leu Leu Val Arg Glu Glu Gly Leu Met Gln
                   405                 410                 415
   Ser Cys Asn Gln Met His Ala Ser Tyr Leu Phe Gln Gln Asp Lys His
               420                 425                 430
   Tyr Asp Leu Ser Tyr Asp Thr Gly Asp Lys Ala Leu Gln Cys Gly Arg
           435                 440                 445
   His Val Asp Val Phe Lys Leu Trp Leu Met Trp Arg Ala Lys Gly Thr
       450                 455                 460
   Thr Gly Phe Glu Ala His Ile Asp Lys Cys Leu Glu Leu Ala Glu Tyr
   465                 470                 475                 480
   Leu Tyr Ser Ile Ile Lys Asn Arg Glu Gly Tyr Glu Met Val Phe Asp
                   485                 490                 495
   Gly Lys Pro Gln His Thr Asn Val Cys Phe Trp Tyr Val Pro Pro Ser
               500                 505                 510
   Leu Arg Val Leu Glu Asp Asn Glu Glu Arg Met Asn Arg Leu Ser Lys
           515                 520                 525
   Val Ala Pro Val Ile Lys Ala Arg Met Met Glu Tyr Gly Thr Thr Met
       530                 535                 540
   Val Ser Tyr Gln Pro Leu Gly Asp Lys Val Asn Phe Phe Arg Met Val
   545                 550                 555                 560
   Ile Ser Asn Pro Ala Ala Thr His Gln Asp Ile Asp Phe Leu Ile Glu
                   565                 570                 575
   Glu Ile Glu Arg Leu Gly Gln Asp Leu
               580                 585
<210> 33
   <211> 1758
   <212> DNA
   <213> Canis lupus
<400> 33
   atggcatctc caggctctgg cttctggtcc ttcgggtctg aagatggctc cggggatccc      60
   gagaacccca gcacagcgag agcctggtgt caggtggccc agaagttcac gggcggcatc     120
   ggaaacaagc tgtgcgccct gctctacgga gatgccgaga agcccgcgga gagtggcggg     180
   agcgagcccc cgcgcgccac ctccaggaag gccgcctgcg cttgtaatca gaagccttgc     240
   agctgcccca aagcggaggt caactatgcg tttctacacg caacagacct gctgccagcc     300
   tgtgatggag aaaggcccac gttggcgttt ctgcaagatg ttatggacat tttgcttcag     360
   tatgttgtga aaagtttcga tagatcaacc aaagtgattg atttccatta ccctaatgag     420
   ctccttcaag agtataactg ggaattggca gaccaaccac aaaatttgga ggaaattttg     480
   atgcattgcc aaacgactct aaaatatgca attaaaacag ggcatcccag atatttcaat     540
   cagctttcca ctggactgga tatggttgga ttagcagcag actggctgac atcaacagca     600
   aacacaaaca tgttcaccta tgaaattgct ccagtatttg tgctcttgga atatgtcaca     660
   ctaaagaaaa tgagagaaat cattggctgg ccgggaggct ctggcgatgg gatattttct       720
   cctggtggcg ctatttctaa catgtatgcc atgctgatcg cacgctttaa gatgttccca       780
   gaagtcaagg agaaaggaat ggctgcggtt cccaggctca ttgccttcac atctgagcat       840
   agtcactttt ctctcaagaa gggagctgca gctttgggga ttggaacaga cagcgtgatt       900
   ctgattaaat gtgatgagag ggggaaaatg gtcccatctg atcttgaaag aaggatcctt       960
   gaagccaaac aaaaaggatt tgttcctttc cttgtgagcg ccacagctgg gaccaccgtg      1020
   tatggagcat tcgaccccct cttagcagtt gctgacattt gtaaaaagta caagatctgg      1080
   atgcatgtgg atgctgcttg gggtggggga ttactgatgt cccggaagca caaatggaag      1140
   ctgagcggcg tggagagggc caactctgtg acatggaacc cacacaagat gatgggcgtc      1200
   cctttacagt gctccgctct cctggttaga gaagagggat tgatgcagag ttgcaaccag      1260
   atgcatgcct cctacctctt ccagcaagat aaacactatg acctgtccta tgatactggg      1320
   gataaggcct tacagtgtgg acgccacgtt gatgttttta aattatggct aatgtggagg      1380
   gcaaagggca ccactgggtt tgaagcacat attgataagt gcctggagct ggctgagtat      1440
   ttatacagta tcataaaaaa ccgagaagga tacgaaatgg tgtttgatgg aaagcctcag      1500
   cacacaaatg tctgcttctg gtacgtgcct ccaagtttgc gtgtcctgga agacaatgaa      1560
   gagagaatga accgcctctc aaaggtggcc ccagtgatta aagcccgaat gatggagtat      1620
   gggaccacaa tggtcagcta tcagcccttg ggagacaagg tcaatttctt ccgcatggtt      1680
   atctcaaatc ccgcagcaac tcaccaagac atcgacttcc tgattgaaga aatagaacgc      1740
   cttggacaag atttataa                                                    1758
<210> 34
   <211> 585
   <212> PRT
   <213> Felis catus
<400> 34
   Met Ala Thr Pro Gly Ser Gly Phe Trp Ser Phe Gly Ser Glu Asp Gly
   1               5                   10                  15
   Ser Gly Asp Pro Glu Asn Pro Gly Thr Ala Arg Ala Trp Cys Gln Val
               20                  25                  30
   Ala Gln Lys Phe Thr Gly Gly Ile Gly Asn Lys Leu Cys Ala Leu Leu
           35                  40                  45
   Tyr Gly Asp Ser Glu Lys Pro Ala Glu Ser Gly Gly Ser Gln Pro Ala
       50                  55                  60
   Arg Ala Thr Ser Arg Lys Ala Thr Cys Ala Cys Asn Gln Lys Pro Cys
   65                  70                  75                  80
   Ser Cys Pro Lys Ala Asp Val Asn Tyr Ala Phe Leu His Ala Thr Asp
                   85                  90                  95
   Leu Leu Pro Ala Cys Asp Gly Glu Arg Pro Thr Leu Ala Phe Leu Gln
               100                 105                 110
   Asp Val Met Gly Ile Leu Leu Gln Tyr Val Val Lys Ser Phe Asp Arg
           115                 120                 125
   Ser Thr Lys Val Ile Asp Phe His Tyr Pro Asn Glu Leu Leu Gln Glu
       130                 135                 140
   Tyr Asn Trp Glu Leu Ala Asp Gln Pro Gln Asn Leu Glu Glu Ile Leu
   145                 150                 155                 160
   Met His Cys Gln Thr Thr Leu Lys Tyr Ala Ile Lys Thr Gly His Pro
                   165                 170                 175
   Arg Tyr Phe Asn Gln Leu Ser Thr Gly Leu Asp Met Val Gly Leu Ala
               180                 185                 190
   Ala Asp Trp Leu Thr Ser Thr Ala Asn Thr Asn Met Phe Thr Tyr Glu
           195                 200                 205
   Ile Ala Pro Val Phe Val Leu Leu Glu Tyr Val Thr Leu Lys Lys Met
       210                 215                 220
   Arg Glu Ile Ile Gly Trp Pro Gly Gly Ser Gly Asp Gly Ile Phe Ser
   225                 230                 235                 240
   Pro Gly Gly Ala Ile Ser Asn Met Tyr Ala Met Leu Ile Ala Arg Phe
                   245                 250                 255
   Lys Met Phe Pro Glu Val Lys Glu Lys Gly Met Ala Ala Val Pro Arg
               260                 265                 270
   Leu Ile Ala Phe Thr Ser Glu His Ser His Phe Ser Leu Lys Lys Gly
           275                 280                 285
   Ala Ala Ala Leu Gly Ile Gly Thr Asp Ser Val Ile Leu Ile Lys Cys
       290                 295                 300
   Asp Glu Arg Gly Lys Met Ile Pro Ser Asp Leu Glu Arg Arg Ile Leu
   305                 310                 315                 320
   Glu Ala Lys Gln Lys Gly Phe Val Pro Phe Leu Val Ser Ala Thr Ala
                   325                 330                 335
   Gly Thr Thr Val Tyr Gly Ala Phe Asp Pro Leu Leu Ala Val Ala Asp
               340                 345                 350
   Ile Cys Lys Lys Tyr Lys Ile Trp Met His Val Asp Ala Ala Trp Gly
           355                 360                 365
   Gly Gly Leu Leu Met Ser Arg Lys His Lys Trp Lys Leu Ser Gly Val
       370                 375                 380
   Glu Arg Ala Asn Ser Val Thr Trp Asn Pro His Lys Met Met Gly Val
   385                 390                 395                 400
   Pro Leu Gln Cys Ser Ala Leu Leu Val Arg Glu Glu Gly Leu Met Gln
                   405                 410                 415
   Ser Cys Asn Gln Met His Ala Ser Tyr Leu Phe Gln Gln Asp Lys His
               420                 425                 430
   Tyr Asp Leu Ser Tyr Asp Thr Gly Asp Lys Ala Leu Gln Cys Gly Arg
           435                 440                 445
   His Val Asp Val Phe Lys Leu Trp Leu Met Trp Arg Ala Lys Gly Thr
       450                 455                 460
   Thr Gly Phe Glu Ala His Ile Asp Lys Cys Leu Glu Leu Ala Glu Tyr
   465                 470                 475                 480
   Leu Tyr Asn Ile Ile Lys Asn Arg Glu Gly Tyr Glu Met Val Phe Asp
                   485                 490                 495
   Gly Lys Pro Gln His Thr Asn Val Cys Phe Trp Tyr Val Pro Pro Ser
               500                 505                 510
   Leu Arg Val Leu Glu Asp Asn Glu Glu Arg Met Ser Arg Leu Ser Lys
           515                 520                 525
   Val Ala Pro Val Ile Lys Ala Arg Met Met Glu Tyr Gly Thr Thr Met
       530                 535                 540
   Val Ser Tyr Gln Pro Leu Gly Asp Lys Val Asn Phe Phe Arg Met Val
   545                 550                 555                 560
   Ile Ser Asn Pro Ala Ala Thr His Gln Asp Ile Asp Phe Leu Ile Glu
                   565                 570                 575
   Glu Ile Glu Arg Leu Gly Gln Asp Leu
               580                 585
<210> 35
   <211> 1758
   <212> DNA
   <213> Felis catus
<400> 35
   atggcaactc caggctcagg cttttggtcc ttcgggtctg aagatggctc cggggatccc 60
   gagaaccccg gcacagcgag agcctggtgt caggtggccc agaagttcac gggcggcatc 120
   ggaaacaagc tgtgcgccct gctctacggg gattcagaga agccggcaga gagtggaggg 180
   agccagcccg cgcgggccac ctcccggaag gccacctgtg cctgtaacca gaagccttgc 240
   agctgcccca aagcggatgt caactatgcg tttctacacg caacagacct gctgccagcc 300
   tgtgatggag aaaggcccac tttggcgttt ctgcaagatg taatgggcat tttgcttcag 360
   tatgtggtga aaagtttcga cagatcaacc aaagtgattg atttccatta ccctaatgag 420
   ctcctgcaag agtataactg ggaattggca gaccaaccac aaaatttgga ggaaattttg 480
   atgcattgcc aaacgactct aaaatatgca ataaaaacag ggcatcccag gtacttcaat 540
   caactttcca cgggactgga tatggttgga ttagcagcag actggctgac atcaacagca 600
   aacactaata tgttcaccta tgaaattgct ccagtatttg tgctcttgga atatgtcaca 660
   ctgaaaaaaa tgagagaaat cattggctgg cctgggggct ccggcgatgg gatattttct 720
   cctggtggcg ctatatctaa catgtatgcc atgctgattg cacgctttaa gatgttccca 780
   gaagtcaagg agaaaggaat ggctgctgtt cccaggctca ttgccttcac atccgagcat 840
   agtcattttt ctctcaagaa gggagctgca gctctgggga ttggaacaga cagcgtgatt 900
   ctgattaaat gcgatgagag agggaaaatg atcccatctg atcttgaaag aaggatcctt 960
   gaagccaaac agaaaggatt tgttcctttc cttgtgagtg ccacagctgg gaccactgtg 1020
   tatggagcat ttgaccccct cttggcggtc gctgacattt gcaaaaagta caagatctgg 1080
   atgcatgtgg atgcagcttg ggtgggggga ttactgatgt cccggaaaca caagtggaaa 1140
   ctgagcggcg tggagagggc caactctgtg acatggaacc cacacaagat gatgggcgtc 1200
   cccttacagt gctctgctct cctggttaga gaagaggggt tgatgcagag ttgcaaccag 1260
   atgcatgctt cctacctttt ccagcaagat aaacactacg acctgtccta cgacactgga 1320
   gacaaggcct tacagtgtgg acgccatgtc gatgttttta aattatggct aatgtggagg 1380
   gcaaagggca ccactgggtt tgaagcacat attgataagt gcttggagct ggcagaatat 1440
   ttatacaata tcataaaaaa ccgagaagga tatgaaatgg tgtttgatgg aaagcctcag 1500
   cacacaaatg tctgcttctg gtacgtgcct ccaagtttgc gagtcctgga agacaatgaa 1560
   gagagaatga gccgcctctc aaaggtggcc ccagtgatta aagccagaat gatggagtat 1620
   gggaccacaa tggtcagcta tcagcccttg ggagacaagg tcaatttctt ccgcatggtc 1680
   atctcaaatc ccgcagcaac tcaccaagac attgacttcc tgattgaaga aatagaacgc 1740
   cttggacaag atttataa 1758
<210> 36
   <211> 585
   <212> PRT
   <213> Equus caballus
<400> 36
   Met Ala Ser Pro Gly Ser Gly Phe Trp Ser Phe Gly Ser Glu Asp Gly
   1               5                   10                  15
   Ser Gly Asp Pro Glu Asn Pro Gly Thr Ala Arg Ala Trp Cys Gln Val
               20                  25                  30
   Ala Gln Lys Phe Thr Gly Gly Ile Gly Asn Lys Leu Cys Ala Leu Leu
           35                  40                  45
   Tyr Gly Asp Ala Glu Lys Ala Ala Glu Ser Gly Gly Ser Glu Pro Pro
       50                  55                  60
   Arg Ala Thr Ser Arg Lys Ala Ala Cys Ser Cys Asn Gln Lys Pro Cys
   65                  70                  75                  80
   Ser Cys Ser Lys Ala Asp Val Asn Tyr Ala Phe Leu His Ala Thr Asp
                   85                  90                  95
   Leu Leu Pro Ala Cys Asp Gly Glu Arg Pro Thr Leu Ala Phe Leu Gln
               100                 105                 110
   Asp Val Met Asp Ile Leu Leu Gln Tyr Val Val Lys Ser Phe Asp Arg
           115                 120                 125
   Ser Thr Lys Val Ile Asp Phe His Tyr Pro Asn Glu Leu Leu Gln Glu
       130                 135                 140
   Tyr Asn Trp Glu Leu Ala Asp Gln Pro Gln Asn Leu Glu Glu Ile Leu
   145                 150                 155                 160
   Met His Cys Gln Thr Thr Leu Lys Tyr Ala Ile Lys Thr Gly His Pro
                   165                 170                 175
   Arg Tyr Phe Asn Gln Leu Ser Thr Gly Leu Asp Met Val Gly Leu Ala
               180                 185                 190
   Ala Asp Trp Leu Thr Ser Thr Ala Asn Thr Asn Met Phe Thr Tyr Glu
           195                 200                 205
   Ile Ala Pro Val Phe Val Leu Leu Glu Tyr Val Thr Leu Lys Lys Met
       210                 215                 220
   Arg Glu Ile Ile Gly Trp Pro Gly Gly Ser Gly Asp Gly Ile Phe Ser
   225                 230                 235                 240
   Pro Gly Gly Ala Ile Ser Asn Met Tyr Ala Met Leu Ile Ala Arg Phe
                   245                 250                 255
   Lys Met Phe Pro Glu Val Lys Glu Lys Gly Met Ala Ala Val Pro Arg
               260                 265                 270
   Leu Ile Ala Phe Thr Ser Glu His Ser His Phe Ser Leu Lys Lys Gly
           275                 280                 285
   Ala Ala Ala Leu Gly Ile Gly Thr Asp Ser Val Ile Leu Ile Arg Cys
       290                 295                 300
   Asp Glu Arg Gly Lys Met Ile Pro Ser Asp Leu Glu Arg Arg Ile Leu
   305                 310                 315                 320
   Glu Ala Lys Gln Lys Gly Phe Val Pro Phe Leu Val Ser Ala Thr Ala
                   325                 330                 335
   Gly Thr Thr Val Tyr Gly Ala Phe Asp Pro Leu Leu Ala Val Ala Asp
               340                 345                 350
   Ile Cys Lys Lys Tyr Lys Ile Trp Met His Val Asp Ala Ala Trp Gly
           355                 360                 365
   Gly Gly Leu Leu Met Ser Arg Lys His Lys Trp Lys Leu Ser Gly Val
       370                 375                 380
   Glu Arg Ala Asn Ser Val Thr Trp Asn Pro His Lys Met Met Gly Val
   385                 390                 395                 400
   Pro Leu Gln Cys Ser Ala Leu Leu Val Arg Glu Glu Gly Leu Met Gln
                   405                 410                 415
   Ser Cys Asn Gln Met His Ala Ser Tyr Leu Phe Gln Gln Asp Lys His
               420                 425                 430
   Tyr Asp Leu Ser Tyr Asp Thr Gly Asp Lys Ala Leu Gln Cys Gly Arg
           435                 440                 445
   His Val Asp Val Phe Lys Leu Trp Leu Met Trp Arg Ala Lys Gly Thr
   450 455 460
   Thr Gly Phe Glu Ala His Ile Asp Lys Cys Leu Glu Leu Ala Glu Tyr
   465 470 475 480
   Leu Tyr Asn Ile Ile Lys Asn Arg Glu Gly Tyr Glu Met Val Phe Asp
   485 490 495
   Gly Lys Pro Gln His Thr Asn Val Cys Phe Trp Tyr Val Pro Pro Ser
   500 505 510
   Leu Arg Val Leu Glu Asp Asn Glu Glu Arg Met Ser Arg Leu Ser Lys
   515 520 525
   Val Ala Pro Val Ile Lys Ala Arg Met Met Glu Tyr Gly Thr Thr Met
   530 535 540
   Val Ser Tyr Gln Pro Leu Gly Asp Lys Val Asn Phe Phe Arg Met Val
   545 550 555 560
   Ile Ser Asn Pro Ala Ala Thr His Gln Asp Ile Asp Phe Leu Ile Glu
   565 570 575
   Glu Ile Glu Arg Leu Gly Gln Asp Leu
   580 585
<210> 37
   <211> 1758
   <212> DNA
   <213> Equus caballus
<400> 37
   atggcatctc ccggctccgg cttttggtcc tttgggtctg aagatggctc cggggatccc      60
   gagaaccctg gcacagcgag agcctggtgt caggtggccc agaagttcac cggcggcatc     120
   ggaaacaagc tatgcgccct gctctacgga gacgccgaga aggcggcgga gagcggcggg     180
   agcgagcccc cgcgggccac ctcccggaag gccgcctgct cctgcaacca gaagccctgc     240
   agctgctcca aagccgatgt caactatgcg tttctacacg caacagactt gctgccagct     300
   tgtgacggag aaagacccac tttggcgttt ctgcaagatg ttatggacat tttgcttcag     360
   tatgtggtga aaagtttcga tagatcaacc aaagtgattg acttccatta ccctaatgag     420
   ctccttcaag agtataattg ggaattggca gaccaaccac aaaatctgga ggaaattttg     480
   atgcattgcc aaacaacttt aaaatatgca attaaaacag ggcatcctag atatttcaat     540
   caactttcca ctggactgga tatggttgga ttagcagcag actggctgac atcaacagca     600
   aacaccaaca tgttcaccta tgaaattgct ccagtattcg tgcttttgga atatgtcaca     660
   ttaaagaaaa tgagagaaat cattggctgg ccaggaggct ctggcgatgg aatattttct     720
   cctggtggcg ccatatctaa catgtatgcc atgctgattg cacgctttaa gatgttccca     780
   gaagtcaagg agaaaggaat ggccgctgtt cccaggctca ttgccttcac gtctgagcat     840
   agtcattttt ctctcaagaa gggagctgca gccttgggga ttggaacaga cagcgtaatt     900
   ctgattagat gtgatgagag ggggaaaatg atcccatcgg atcttgaaag aagaatcctt     960
   gaagccaaac aaaaaggatt tgtccctttt cttgtgagtg ccacggctgg gaccaccgtg    1020
   tatggagcat tcgatcccct cttagctgtc gctgacattt gcaaaaagta caagatctgg    1080
   atgcatgtgg atgcagcttg gggcggggga ttactgatgt cccggaaaca caagtggaaa    1140
   ctgagtggcg tggagagggc caactctgtg acatggaatc cacacaagat gatgggtgtc    1200
   cctttgcagt gctctgctct cctggttaga gaagagggat tgatgcagag ttgcaaccag    1260
   atgcatgcct cctacctctt tcagcaagat aaacactatg acctgtccta tgacactgga    1320
   gacaaggcct tgcagtgcgg acgccacgtg gatgttttta agttatggct catgtggagg    1380
   gcaaagggaa caactgggtt tgaagcacat attgataagt gtttggagtt ggcggagtat    1440
   ttatacaata tcataaaaaa ccgagaagga tatgaaatgg tgtttgacgg aaagcctcag    1500
   cacaccaatg tctgcttctg gtatgtacct ccgagtctgc gtgttctaga agacaatgaa    1560
   gagagaatga gccgcctctc aaaggtggcc ccggtgatta aagccagaat gatggagtat    1620
   gggaccacaa tggtcagcta ccagcccttg ggagacaagg tcaatttctt ccgcatggtc    1680
   atctcaaatc ccgcagcaac tcaccaagac attgacttcc tgattgaaga aatagaacgc    1740
   cttggacaag atttataa                                                  1758
<210> 38
   <211> 181
   <212> PRT
   <213> Canis lupus
<400> 38
   Met His Gly Ser Ala Leu Leu Cys Cys Cys Leu Val Leu Leu Ala Gly
   1               5                   10                  15
   Val Gly Ala Ser Arg His Gln Ser Thr Leu Leu Glu Asp Asp Cys Thr
               20                  25                  30
   His Phe Pro Ala Ser Leu Pro His Met Leu Arg Glu Leu Arg Ala Ala
           35                  40                  45
   Phe Gly Arg Val Lys Ile Phe Phe Gln Met Lys Asp Lys Leu Asp Asn
       50                  55                  60
   Ile Leu Leu Thr Gly Ser Leu Leu Glu Asp Phe Lys Ser Tyr Leu Gly
   65                  70                  75                  80
   Cys Gln Ala Leu Ser Glu Met Ile Gln Phe Tyr Leu Glu Glu Val Met
                   85                  90                  95
   Pro Arg Ala Glu Asn His Asp Pro Asp Ile Lys Asn His Val Asn Ser
               100                 105                 110
   Leu Gly Glu Lys Leu Lys Thr Leu Arg Leu Arg Leu Arg Leu Arg Arg
           115                 120                 125
   Cys His Arg Phe Leu Pro Cys Glu Asn Lys Ser Lys Ala Val Glu Gln
       130                 135                 140
   Val Lys Ser Ala Phe Ser Lys Leu Gln Glu Lys Gly Val Tyr Lys Ala
   145                 150                 155                 160
   Met Ser Glu Phe Asp Ile Phe Ile Asn Tyr Ile Glu Thr Tyr Met Thr
                   165                 170                 175
   Met Arg Met Lys Ile
               180
<210> 39
   <211> 546
   <212> DNA
   <213> Canis lupus
<400> 39
   atgcatggct cagcactgct ctgttgctgc ctggtcctcc tggccggggt gggagccagc        60
   cgacaccaga gcaccctact tgaggacgac tgcacccact tcccagccag cctgccccac       120
   atgctccgag agctccgagc tgccttcggg agggtgaaga tcttctttca aatgaaggac       180
   aagctggaca acatactgct gaccgggtcc ctgctggagg actttaagag ttacctgggt       240
   tgccaagccc tgtcggagat gatccagttt tacttggagg aggtgatgcc ccgggctgag       300
   aaccacgacc cagacatcaa gaaccacgtg aactccctgg gagagaagct caagaccctc       360
   aggctgagac tgaggctgcg acgctgtcac cgatttcttc cctgtgagaa taagagcaag       420
   gcggtggagc aggtgaagag cgcatttagt aagctccagg agaaaggtgt ctacaaagcc       480
   atgagtgagt ttgacatctt catcaactac atagaaacct acatgacaat gaggatgaaa       540
   atctga                                                                  546
<210> 40
   <211> 178
   <212> PRT
   <213> Felis catus
<400> 40
   Met His Ser Ser Ala Leu Leu Cys Phe Leu Val Phe Leu Ala Gly Val
   1               5                   10                  15
   Gly Ala Ser Arg His Gln Ser Thr Leu Ser Glu Asp Asn Cys Thr His
               20                  25                  30
   Phe Ser Val Ser Leu Pro His Met Leu Arg Glu Leu Arg Ala Ala Phe
           35                  40                  45
   Gly Lys Val Lys Thr Phe Phe Gln Thr Lys Asp Glu Leu His Ser Ile
       50                  55                  60
   Leu Leu Thr Arg Ser Leu Leu Glu Asp Phe Lys Gly Tyr Leu Gly Cys
   65                  70                  75                  80
   Gln Ala Leu Ser Glu Met Ile Gln Phe Tyr Leu Glu Glu Val Met Pro
                   85                  90                  95
   Gln Ala Glu Asn Glu Asp Pro Asp Ile Lys Gln His Val Asn Ser Leu
                   100                 105                 110
   Gly Glu Lys Leu Lys Thr Leu Arg Leu Arg Leu Arg Arg Cys His Arg
           115                 120                 125
   Phe Leu Pro Cys Glu Asn Lys Ser Lys Val Val Glu Gln Val Lys Ser
       130                 135                 140
   Thr Phe Ser Lys Leu Gln Glu Lys Gly Val Tyr Lys Ala Met Gly Glu
   145                 150                 155                 160
   Phe Asp Ile Phe Ile Asn Tyr Ile Glu Ala Tyr Met Thr Met Lys Met
                   165                 170                 175
   Lys Ile
<210> 41
   <211> 537
   <212> DNA
   <213> Felis catus
<400> 41
   atgcacagct cagcacttct gtgtttcctg gtcttcctgg ccggggtagg agccagccga      60
   caccagagca ccctgtctga ggacaactgc acccacttct cagtcagcct gccccacatg     120
   ctccgagagc tccgagctgc cttcggcaag gtgaagactt tctttcaaac caaggacgag     180
   ctgcacagca tattgttgac caggtccttg ctggaggact ttaagggtta cctgggttgc     240
   caagccttgt ccgagatgat ccagttttat ttggaggagg tgatgcccca ggctgagaac     300
   gaggacccag acatcaaaca gcacgtgaac tccctgggag aaaagctgaa gaccctccgg     360
   ctgagactgc ggcgctgtca tcgatttctg ccctgtgaaa acaagagcaa ggtggtggag     420
   caggtgaaga gtacctttag taagctccaa gagaaaggtg tctacaaagc catgggtgag     480
   tttgacatct tcatcaacta catagaagct tacatgacaa tgaagatgaa aatctga        537
<210> 42
   <211> 178
   <212> PRT
   <213> Equus caballus
<400> 42
   Met His Ser Ser Ala Leu Leu Cys Tyr Leu Val Phe Leu Ala Gly Val
   1 5 10 15
   Gly Ala Ser Arg Asp Arg Gly Thr Gln Ser Glu Asn Ser Cys Thr His
   20 25 30
   Phe Pro Thr Ser Leu Pro His Met Leu His Glu Leu Arg Ala Ala Phe
   35 40 45
   Ser Arg Val Lys Thr Phe Phe Gln Met Lys Asp Gln Leu Asp Asn Met
   50 55 60
   Leu Leu Asn Gly Ser Leu Leu Glu Asp Phe Lys Gly Tyr Leu Gly Cys
   65 70 75 80
   Gln Ala Leu Ser Glu Met Ile Gln Phe Tyr Leu Glu Glu Val Met Pro
   85 90 95
   Gln Ala Glu Asn His Gly Pro Asp Ile Lys Glu His Val Asn Ser Leu
   100 105 110
   Gly Glu Lys Leu Lys Thr Leu Arg Val Arg Leu Arg Arg Cys His Arg
   115 120 125
   Phe Leu Pro Cys Glu Asn Lys Ser Lys Ala Val Glu Gln Val Lys Ser
   130 135 140
   Ala Phe Ser Lys Leu Gln Glu Lys Gly Val Tyr Lys Ala Met Ser Glu
   145 150 155 160
   Phe Asp Ile Phe Ile Asn Tyr Ile Glu Ala Tyr Met Thr Thr Lys Met
   165 170 175
   Lys Asn
<210> 43
   <211> 537
   <212> DNA
   <213> Equus caballus
<400> 43
   atgcacagct cagcactgct atgttacctg gtcttcctgg ccggggtggg agccagccga 60
   gaccggggca cccagtctga gaacagctgc acccacttcc caaccagcct gccccacatg 120
   ctccatgagc tccgagccgc cttcagcagg gtgaagactt tctttcaaat gaaggaccag 180
   ctggacaaca tgttgttgaa cgggtccctg ctggaggact ttaagggtta cctgggttgc 240
   caagccttgt cggagatgat ccagttttac ctggaggagg tgatgcccca ggctgagaac 300
   cacggcccag acatcaagga gcacgtgaac tccctggggg aaaagctgaa gaccctccga 360
   gtgaggctgc ggcgctgtca tcgatttctg ccctgtgaaa ataagagcaa ggcagtggag 420
   caggtgaaga gtgccttcag taagctccaa gagaaaggtg tctacaaagc catgagtgag 480
   tttgacatct tcatcaacta catagaagcc tatatgacaa cgaagatgaa aaactga 537
<210> 44
   <211> 185
   <212> PRT
   <213> Canis lupus
<400> 44
   Met Lys Leu Trp Asp Val Val Ala Val Cys Leu Val Leu Leu His Thr
   1               5                   10                  15
   Ala Ser Ala Leu Pro Leu Pro Ala Ala Asn Val Pro Glu Asp Tyr Ser
               20                  25                  30
   Asp Gln Phe Asp Asp Val Met Asp Phe Ile Gln Ala Thr Ile Arg Arg
           35                  40                  45
   Leu Lys Arg Ser Pro Glu Lys Gln Met Ala Val Pro Ala Arg Arg Glu
       50                  55                  60
   Arg Asn Arg Gln Ala Ala Ala Ala Gly Pro Glu His Ser Arg Gly Lys
   65                  70                  75                  80
   Gly Arg Arg Gly Pro Arg Gly Arg Asn Arg Gly Cys Val Leu Thr Ala
                   85                  90                  95
   Ile His Leu Asn Val Thr Asp Leu Gly Leu Gly Tyr Glu Thr Lys Glu
               100                 105                 110
   Glu Leu Ile Phe Arg Tyr Cys Ser Gly Ser Cys Asp Ala Ala Glu Thr
           115                 120                 125
   Met Tyr Asp Lys Ile Leu Lys Asn Leu Ser Lys Ser Arg Arg Leu Ala
       130                 135                 140
   Ser Asp Lys Ala Gly Gln Ala Cys Cys Arg Pro Ile Ala Tyr Asp Asp
   145                 150                 155                 160
   Asp Leu Ser Phe Leu Asp Asp Asn Leu Val Tyr His Ile Leu Arg Lys
                   165                 170                 175
   His Ser Ala Lys Arg Cys Gly Cys Ile
               180                 185
<210> 45
   <211> 558
   <212> DNA
   <213> Canis lupus
<400> 45
   atgaagttat gggatgtcgt ggctgtctgc ctggtgctgc tccacaccgc gtccgccctc          60
   ccgctgcccg ccgcaaacgt gccggaggac tattctgatc agtttgatga cgtcatggat         120
   tttattcagg ccaccatcag aaggctgaaa aggtcacccg agaaacaaat ggccgtgcca         180
   gcgagacgag agcggaatcg tcaggccgcg gccgccggcc cggaacattc cagggggaag        240
   gggcggcgag gcccgagggg cagaaaccgg ggttgtgtct tgactgcgat acatttaaac         300
   gtcactgacc tgggcttggg ctacgaaacc aaggaggaac tgatttttag gtactgcagc         360
   ggctcctgcg acgcggccga gaccatgtac gacaaaatat taaaaaactt atccaaaagt         420
   agaaggctgg cgagtgacaa agcagggcag gcttgctgca gacccatcgc ctacgatgac         480
   gacctgtcgt ttttagatga caacctggtt taccatattc taagaaagca ttccgctaaa         540
   aggtgtggat gtatctga                                                       558
<210> 46
   <211> 211
   <212> PRT
   <213> Felis catus
<400> 46
   Met Lys Leu Trp Asp Val Val Ala Val Cys Leu Val Leu Leu His Thr
   1               5                   10                  15
   Ala Ser Ala Phe Pro Leu Pro Ala Gly Lys Arg Pro Pro Glu Ala Pro
               20                  25                  30
   Ala Glu Asp Arg Ser Leu Gly Arg Arg Arg Ala Pro Phe Ala Leu Ser
           35                  40                  45
   Ser Asp Ser Asn Met Pro Glu Asp Tyr Pro Asp Gln Phe Asp Asp Val
       50                  55                  60
   Met Asp Phe Ile Gln Ala Thr Ile Arg Arg Leu Lys Arg Ser Pro Glu
   65                  70                  75                  80
   Lys Gln Met Ala Leu Pro Pro Arg Arg Glu Arg Asn Arg Gln Ala Ala
                   85                  90                  95
   Ala Ala Asn Pro Glu Asn Ser Arg Gly Lys Gly Arg Arg Gly Gln Arg
               100                 105                 110
   Gly Arg Asn Arg Gly Cys Val Leu Thr Ala Ile His Leu Asn Val Thr
           115                 120                 125
   Asp Leu Gly Leu Gly Tyr Glu Thr Lys Glu Glu Leu Ile Phe Arg Tyr
       130                 135                 140
   Cys Ser Gly Ser Cys Asp Ala Ala Glu Thr Met Tyr Asp Lys Ile Leu
   145                 150                 155                 160
   Lys Asn Leu Ser Lys Asn Arg Arg Leu Val Ser Asp Lys Val Gly Gln
                   165                 170                 175
   Ala Cys Cys Arg Pro Ile Ala Tyr Asp Asp Asp Leu Ser Phe Leu Asp
               180                 185                 190
   Asp Asn Leu Val Tyr His Ile Leu Arg Lys His Ser Ala Lys Arg Cys
           195                 200                 205
   Gly Cys Ile
       210
<210> 47
   <211> 636
   <212> DNA
   <213> Felis catus
<400> 47
   atgaagttat gggatgtcgt ggctgtctgc ctggtgctgc tccacaccgc gtccgccttc      60
   ccgctgcccg ccggtaagag gcctcccgag gcgcccgccg aagaccgctc cctcggccgc     120
   cgccgcgcgc ccttcgcgct gagcagtgac tcaaatatgc cagaggatta tcctgatcag     180
   tttgacgacg tcatggattt tattcaagct accatcagaa gactgaaaag gtcacccgag     240
   aaacaaatgg ccttgccgcc tagaagagag cggaatcggc aggcggcggc cgccaacccg     300
   gagaattcca gagggaaagg tcggcgaggc cagaggggca gaaatcgggg ttgtgtctta     360
   actgcgatac atttgaacgt caccgacctg ggtttgggct acgaaaccaa ggaggaactg     420
   atttttaggt actgcagcgg ctcctgtgat gcagctgaga caatgtacga caaaatatta     480
   aaaaacttat ccaaaaacag aaggctggtg agtgacaaag tcgggcaggc atgttgcaga     540
   cccatcgcct atgacgacga cctgtcgttt ttagatgaca acctggttta ccatattcta     600
   agaaagcatt ccgctaaaag gtgtggatgt atctga                               636
<210> 48
   <211> 185
   <212> PRT
   <213> Equus caballus
<400> 48
   Met Lys Leu Trp Asp Val Val Ala Val Cys Leu Val Leu Leu His Thr
   1               5                   10                  15
   Ala Ser Ala Phe Pro Leu Pro Ala Ala Asn Met Pro Glu Asp Tyr Pro
               20                  25                  30
   Asp Gln Phe Asp Asp Val Met Asp Phe Ile Gln Ala Thr Ile Lys Arg
           35                  40                  45
   Leu Lys Arg Ser Pro Asp Lys Gln Met Ala Val Leu Pro Arg Arg Glu
       50                  55                  60
   Arg Asn Arg Gln Ala Ala Ala Ala Asn Pro Glu Asn Ser Arg Arg Lys
   65                  70                  75                  80
   Gly Gln Arg Gly Gln Arg Gly Lys Asn Arg Gly Cys Val Leu Thr Ala
                   85                  90                  95
   Ile His Leu Asn Val Thr Asp Leu Gly Leu Gly Tyr Glu Thr Lys Glu
               100                 105                 110
   Glu Leu Ile Phe Arg Tyr Cys Ser Gly Ser Cys Glu Ala Ala Glu Thr
           115                 120                 125
   Met Tyr Asp Lys Ile Leu Lys Asn Leu Ser Lys Asn Arg Arg Leu Val
       130                 135                 140
   Ser Asp Lys Val Gly Gln Ala Cys Cys Arg Pro Ile Ala Phe Asp Asp
   145                 150                 155                 160
   Asp Leu Ser Phe Leu Asp Asp Asn Leu Val Tyr His Ile Leu Arg Lys
                   165                 170                 175
   His Ser Ala Lys Arg Cys Gly Cys Ile
               180                 185
<210> 49
   <211> 558
   <212> DNA
   <213> Equus caballus
<400> 49
   atgaagttat gggatgtcgt ggctgtctgc ctggtgctgc tccacaccgc gtccgccttc      60
   ccgctgcccg ccgcaaatat gccagaggat tatcctgatc agtttgatga tgtcatggat     120
   tttattcaag ccaccattaa aagactgaaa aggtcaccag ataaacaaat ggcagtgctt     180
   cctagaagag agcggaatcg gcaggctgca gctgccaacc cggagaattc cagaaggaaa     240
   ggtcagcgag gccagagggg caaaaaccgg ggttgtgtct taaccgcgat acatttaaat        300
   gtcactgact tgggtttggg ctacgaaacc aaggaggaac tgatttttag gtactgcagt        360
   ggctcctgcg aggcagccga gacaatgtac gacaaaatat taaaaaactt atccaaaaat        420
   agaaggctgg tgagtgacaa agtagggcag gcatgttgca gacccatcgc cttcgatgac        480
   gacctgtcat ttttagatga taacttggtt taccatattc taagaaagca ttccgctaaa        540
   aggtgtggat gtatctga                                                      558